# EUROPÄISCHE PATENTSCHRIFT

(11) **EP 2 672 874 B1**
(45) Veröffentlichungstag und Bekanntmachung des Hinweises auf die Patenterteilung: **30.03.2022**
(21) Anmeldenummer: 12702806.6
(22) Anmeldetag: 01.02.2012
(51) Int. Cl.: A47L 15/24, A47L 15/00, A47L 15/42, A47J 43/24, A61G 9/02, A61L 2/14

(54) **REINIGUNGSVORRICHTUNG ZUR REINIGUNG VON REINIGUNGSGUT**
CLEANING APPARATUS FOR CLEANING ARTICLES
DISPOSITIF DE NETTOYAGE POUR NETTOYER DES OBJETS À NETTOYER

(30) Priorität: 08.02.2011 DE 102011003782
(43) Veröffentlichungstag der Anmeldung: 18.12.2013
(73) Patentinhaber: MEIKO Maschinenbau GmbH & Co. KG, 77652 Offenburg (DE)
(72) Erfinder: SCHNEIDER, Vera, 77652 Offenburg (DE); NÄGER, Thomas, 77652 Offenburg (DE); PEUKERT, Thomas, 77815 Bühl (Baden) (DE); RAUBER, Hans-Josef, 77784 Oberharmersbach (DE); WIEGAND, Ingo, 77830 Bühlerbach (DE)
(74) Vertreter: Altmann Stößel Dick Patentanwälte PartG mbB
(86) Internationale Anmeldenummer: PCT/EP2012/051643
(87) Internationale Veröffentlichungsnummer: WO 2012/107332

(56) Entgegenhaltungen:
- WO-A1-2008/145217
- WO-A1-2011/141263
- WO-A2-98/30249
- WO-A2-2005/018407
- DE-A1- 19 858 344
- FR-A1- 2 827 777
- JP-A- 2005 103 376
- JP-A- 2010 194 060
- JP-A- 2010 220 741
- US-A1- 2008 236 631
- US-A1- 2010 226 821

## Beschreibung

### Gebiet der Erfindung

Die Erfindung betrifft eine Reinigungsvorrichtung und ein Verfahren zur Reinigung von Reinigungsgut. Die Reinigungsvorrichtung und das Verfahren können eingesetzt werden im Bereich der Reinigung von Reinigungsgut in Form von Pflegeutensilien wie beispielsweise Steckbecken, Bettpfannen, Urinflaschen oder ähnlichen Pflegeutensilien, welche mit menschlichen oder tierischen Ausscheidungen in Berührung kommen.

### Stand der Technik

Aus dem Stand der Technik sind eine Vielzahl von Reinigungsvorrichtungen zur Reinigung unterschiedlicher Arten von Reinigungsgut bekannt. Beispielsweise sind derartige Reinigungsvorrichtungen aus DE 10 2004 056 052 A1 in Form von Geschirrspülmaschinen bekannt, insbesondere für den gewerblichen Einsatz. Ebenfalls beschrieben werden Reinigungsvorrichtungen in Form sogenannter Reinigungs- und Desinfektionsgeräte, mittels derer Pflegeutensilien für den Pflegebedarf gereinigt werden können. Aus DE 10 2006 050 876 A1 sind beispielsweise Geschirrspülmaschinen, insbesondere für den gewerblichen Einsatz, bekannt. Aus DE 10 2006 039 434 A1 sind ebenfalls Geschirrspülmaschinen bekannt, welche als Durchlauf-Geschirrspülmaschinen und insbesondere als Mehrtankgeschirrspülmaschinen ausgestaltet sind. Weitere Arten von Reinigungsvorrichtungen in Form von Reinigungs- und Desinfektionsgeräten sind beispielsweise aus DE 103 48 344 B4 oder aus EP 1 824 373 B1 bekannt. Dabei offenbart beispielsweise DE 103 48 344 B4 ein Verfahren und eine Vorrichtung zur Rückkühlung von Reinigungsgut in Reinigungs- und Desinfektionsautomaten für menschliche Ausscheidungen aufnehmende Behälter. Innerhalb einer Kammer erfolgt ein Abspülen des in dieser enthaltenen Reinigungsgutes, woran sich ein Vorreinigungsspülschritt anschließt. Danach wird das in der Kammer enthaltene Reinigungsgut mit einem einen Klarspülzusatz enthaltenen Wasser endgereinigt, bevor ein Desinfektionsschritt des Reinigungsgutes in der Kammer durch Einleitung von Wasserdampf in diese erfolgt. In die mit Wasserdampf befüllte Kammer wird zwangsweise Luft bei geschlossener Tür eingebracht, wodurch ein Niederschlag von Wasserdampf innerhalb der Kammer sowie eine Abkühlung und eine Trocknung des in der Kammer enthaltenen Reinigungsgutes bewirkt wird. Die Kammer umfasst an ihrem unteren Ende einen einen Siphonbogen enthaltenden Ablauf, über welchen die Reste menschlicher Ausscheidungen in ein Abwassersystem eingeleitet werden können. Zur Vermeidung von Geruchsbildung in der Kammer dient der im Ablauf ausgebildete Siphonbogen, der im Allgemeinen als Geruchssperre in Abwassersystemen vorgesehen ist. Ferner wird über eine Abluftleitung Luft aus der Kammer in den Ablauf geführt, wobei die Mündungsstelle der Abluftleitung hinter dem Siphonbogen des Ablaufs liegt.

Aus dem oben beschrieben Stand der Technik ist es weiterhin bekannt, dass der Sicherstellung einer Desinfektion des Reinigungsguts eine zunehmende Bedeutung beigemessen wird. Insbesondere in hygienekritischen Bereichen wie Krankenhäusern oder Pflegeeinrichtungen, jedoch auch in Bereichen einer Gemeinschaftsverpflegung, insbesondere in Großküchen, muss eine Hygienisierung des Reinigungsguts sichergestellt werden.

Aus dem Stand der Technik sind Desinfektionsprozesse in Reinigungsvorrichtungen, beispielsweise in Geschirrspülmaschinen oder Reinigungs- und Desinfektionsautomaten, in der Regel als thermische Desinfektionsschritte bekannt. Eine derartige thermische Desinfektion wird in der Regel mittels heißer wässriger Flüssigkeiten und/oder mittels Dampf durchgeführt. Das hierbei verwendete Desinfektionsmedium wird entweder ungerichtet in eine Reinigungskammer eingebracht und gibt seinen Wärmeinhalt auf das Reinigungsgut ab, oder das Medium wird direkt auf das Reinigungsgut aufgesprüht, wobei ebenfalls ein Wärmeinhalt abgegeben wird. Dies ist beispielsweise in den oben genannten DE 103 48 344 B4 und EP 1 824 373 B1 sowie in DE 10 2004 056 052 A1 beschrieben. Für Geschirrspülmaschinen sind derartige thermische Desinfektionsprozesse beispielsweise ebenfalls in DE 10 2004 056 052 A1 oder, für Durchlaufgeschirrspülmaschinen, in DE 10 2006 039 434 A1 beschrieben.

Weiterhin sind auch Desinfektionsverfahren bekannt und weitverbreitet, welche mit chemischen Mitteln durchgeführt werden, sowie Kombinationen thermischer und chemischer Desinfektionsverfahren. Aus DE 10 2006 050 876 A1 sind weiterhin ein Verfahren und eine Vorrichtung bekannt, bei denen eine Desinfektionswirkung durch einen kombinierten Einsatz von UV-Licht und/oder durch den Einsatz von durch einen UV-Strahler erzeugtem Ozon erreicht wird.

Die aus dem Stand der Technik bekannten Vorrichtungen und Verfahren weisen eine Reihe technischer Herausforderungen und Nachteile auf. Ein Nachteil insbesondere der thermischen Desinfektionsverfahren besteht in einem relativ hohen Energieeinsatz zum Aufheizen der für die Desinfektion verwendeten Medien. So erfolgt beispielsweise in vielen Fällen eine Aufheizung flüssiger Medien auf beispielsweise 90°C. Alternativ oder zusätzlich muss als Desinfektionsmedium in vielen Fällen Wasserdampf erzeugt werden. Mit einem erhöhten Energieeinsatz geht in der Regel auch eine relativ lange Prozessdauer einher. Ein weiterer Nachteil besteht darin, dass am Ende des Desinfektionsvorgangs die Reinigungsgüter in der Regel sehr heiß sind und erst wieder abgekühlt werden müssen, damit sich ein Bedienpersonal nicht an dem heißen Reinigungsgut verletzen kann.

Chemische Desinfektionsverfahren arbeiten hingegen mit in der Regel aggressiven Chemikalien. Diese können während der Verwendung und/oder bei einer Entsorgung ökologische Probleme bereiten oder gesundheitliche Risiken darstellen. Zudem ist bei vielen chemischen Desinfektionsverfahren, die auf Geschirr und ähnliche Gegenstände, die zur Zubereitung, Darreichung oder Aufbewahrung von Speisen eingesetzt werden, eine geschmackliche Belastung des Reinigungsguts festzustellen. Insbesondere bei der Reinigung von Trinkgläsern rufen beispielsweise chemische Desinfektionsmittel in vielen Fällen einen anhaftenden Chlorgeschmack und/oder Chlorgeruch hervor, welcher unerwünscht ist.

Insbesondere aus dem medizinischen Bereich sind weiterhin Plasmaquellen für Desinfektionszwecke bekannt. So ist beispielsweise aus WO 2010/094304 A1 eine Elektrodenanordnung zur Erzeugung eines nicht-thermischen Plasmas bekannt. Diese umfasst eine schichtförmige erste Elektrode, die aus einem elektrisch leitfähigen Material hergestellt ist, eine schichtförmige zweite Elektrode, die aus einem elektrisch leitfähigen Material hergestellt ist, wobei die zweite Elektrode von der ersten Elektrode elektrisch isoliert ist. Weiterhin ist eine dielektrische Barriere zwischen der ersten Elektrode und der zweiten Elektrode angeordnet, sodass das nicht-thermische Plasma durch eine dielektrische Barrieren-Entladung erzeugt wird. Mindestens eine der Elektroden umfasst eine Mehrzahl von Perforationen, die über die Elektrode verteilt sind. EP 2 170 022 A1 und WO 2010/034451 A1 beschreiben eine Vorrichtung zum Applizieren eines Plasmas beispielsweise auf menschlicher Haut. WO 2010/094307 A1 beschreibt eine Vorrichtung zur Plasmabehandlung von Körperteilen, und EP 2 160 081 A1 beschreibt eine Vorrichtung zum Applizieren eines Plasmas, bei der dem Plasma zusätzliche Stoffe beigefügt werden, um die Wirkung zu beeinflussen. In EP 1 993 329 A1 und in EP 1 765 044 A1 werden weitere Ausführungen von Plasmaquellen beschrieben.

In DE 102007037984 A1 wird ein Verfahren zur Textilreinigung und Textildesinfektion mittels Plasma beschrieben. Auch aus Verfahrenstechnik 1-2/2010, Seite 12, ist eine Plasmaquelle zur Entkeimung von PET-Flaschen bekannt. Hierbei wird ein Stift eingesetzt, welcher einen Plasmajet erzeugt. Mittels dieses Plasmajets können beispielsweise Kunststofffolien und/oder andere Arten von Verpackungen behandelt und auf diese Weise entkeimt werden.

US 2008/0236631 A1 beschreibt ein Verfahren und eine Vorrichtung zum automatischen Reinigen und Dekontaminieren von medizinischen Instrumenten. Unter anderem wird dabei ein Ausspülen der Instrumente in einer Reinigungskammer offenbart. Anschließend kann ein Reinigen mittels eines atmosphärischen Plasmas erfolgen.

### Aufgabe der Erfindung

Es ist daher eine Aufgabe der vorliegenden Erfindung, eine Reinigungsvorrichtung und ein Verfahren zur Reinigung von Reinigungsgut vorzuschlagen, welche die Nachteile bekannter Vorrichtungen und Verfahren zumindest weitgehend vermeiden. Insbesondere soll eine Desinfektion des Reinigungsguts ermöglicht werden, welche schonend für das Reinigungsgut, das Bedienpersonal und die Umwelt ist und welche kostengünstig und ohne größeren Energieaufwand realisierbar ist.

### Offenbarung der Erfindung

Diese Aufgabe wird gelöst durch eine Reinigungsvorrichtung mit den Merkmalen des Anspruchs 1 und ein Verfahren zur Reinigung von Reinigungsgut mit den Merkmalen des Anspruchs 13. Vorteilhafte Weiterbildungen der Erfindung, welche einzeln oder in beliebiger Kombination realisierbar sind, sind in den abhängigen Ansprüchen dargestellt. In einem ersten Aspekt der vorliegenden Erfindung wird eine Reinigungsvorrichtung zur Reinigung von Reinigungsgut vorgeschlagen. Unter einer Reinigungsvorrichtung ist im Rahmen der vorliegenden Erfindung eine Vorrichtung zu verstehen, welche eingerichtet ist, um in dem Reinigungsgut enthaltene Verunreinigung und/oder an dem Reinigungsgut anhaftende Verunreinigungen zumindest weitgehend zu beseitigen und vorzugsweise weiterhin eine Desinfektion des Reinigungsguts durchzuführen, also zumindest einen großen Prozentsatz, beispielsweise mindestens 99%, vorzugsweise mindestens 99,9% oder sogar mindestens 99,999% oder mehr, von an dem Reinigungsgut anhaftenden mikrobiellen Verunreinigungen abzutöten.

Die Reinigungsvorrichtung kann insbesondere mindestens eine Aufnahme und/oder mindestens eine Halterung zur Fixierung und/oder Aufnahme des Reinigungsguts aufweisen, insbesondere mindestens einen Korb, mindestens eine Tasche oder mindestens eine andere Art von Halterung.

Das Reinigungsgut umfasst Pflegeutensilien. Der Begriff der Pflegeutensilien ist weit zu fassen und kann insbesondere Gegenstände umfassen, ausgewählt aus der Gruppe bestehend aus Steckbecken, Bettpfannen, Urinflaschen und Waschschüsseln.

Die Reinigungsvorrichtung weist eine Reinigungskammer zur Aufnahme des Reinigungsguts auf. Unter einer Reinigungskammer ist dabei ein durch ein Gehäuse abgeschlossener Raum zu verstehen, innerhalb dessen das Reinigungsgut zumindest zeitweise aufgenommen und einem Reinigungsverfahren unterzogen werden kann. Beispielsweise kann die Reinigungskammer vollständig oder teilweise durch ein Gehäuse umschlossen sein, insbesondere ein Metallgehäuse. Die Reinigungskammer kann vollständig abgeschlossen sein, wobei beispielsweise ein Druckausgleich mit einer Umgebung erfolgen kann, wobei jedoch beispielsweise ein Einbringen und Austragen von Reinigungsgut durch mindestens eine verschließbare Öffnung, beispielsweise eine Tür, erfolgen kann. Alternativ oder zusätzlich kann die Reinigungskammer jedoch, wie unten noch näher ausgeführt wird, auch als einseitig oder mehrseitig geöffnete Kammer ausgestaltet sein, beispielsweise als Reinigungstunnel, in welche auf einer Seite das Reinigungsgut eingefahren wird, wobei ein Austrag aus der Reinigungskammer beispielsweise auf einer gegenüberliegenden Seite erfolgen kann. Verschiedene andere Ausgestaltungen sind möglich. Die Reinigungskammer kann eingerichtet sein, um innerhalb der Reinigungskammer einen Normaldruck aufrechtzuerhalten und/oder einen Umgebungsdruck, wobei beispielsweise ein Druckausgleich zwischen einem Inneren der Reinigungskammer und einer Umgebung erfolgt. Alternativ oder zusätzlich kann jedoch auch in einem oder mehreren Programmschritten innerhalb der Reinigungskammer ein von einem Umgebungsdruck abweichender Druck herrschen, beispielsweise ein Unterdruck oder ein Überdruck, und/oder es kann innerhalb der Reinigungskammer eine von einer Umgebung abweichende Zusammensetzung einer Atmosphäre vorherrschen. Verschiedene Ausgestaltungen sind möglich.

Die Reinigungsvorrichtung weist in der Reinigungskammer mindestens eine Fluidquelle zur Beaufschlagung des Reinigungsguts mit mindestens einem Reinigungsfluid auf. Das Reinigungsfluid kann insbesondere eine Reinigungsflüssigkeit sein oder umfassen. Alternativ oder zusätzlich sind jedoch auch andere flüssige und/oder gasförmige Medien als Reinigungsfluid einsetzbar. Insbesondere kann das Reinigungsfluid mindestens ein wässriges Reinigungsfluid aufweisen, gegebenenfalls unter Zusatz von Zusatz- und/oder Hilfsstoffen, beispielsweise mindestens einer Reinigerlösung und/oder einer Klarspüllösung und/oder einem Desinfektionsmittel. Verschiedene Ausgestaltungen sind aus dem Bereich der Spültechnik und/oder Reinigungstechnik grundsätzlich bekannt und ebenfalls im Rahmen der vorliegenden Erfindung einsetzbar. Die mindestens eine Fluidquelle kann beispielsweise mindestens eine Düse und/oder mindestens ein Düsensystem aufweisen, mittels dessen das Reinigungsfluid auf das Reinigungsgut aufgesprüht, aufgestrahlt, aufgetropft, aufgegossen oder auf andere Weise aufgebracht wird. Dieses Aufbringen kann drucklos oder auch mittels eines Überdrucks erfolgen.

Zur Lösung der oben beschriebenen Problematik wird vorgeschlagen, die Reinigungsvorrichtung derart auszugestalten, dass diese weiterhin mindestens eine Plasmaquelle aufweist. Die Plasmaquelle ist eingerichtet, um mindestens ein Plasma in mindestens einem Gas zu zünden und mindestens ein Reaktivgas zu erzeugen. Die Reinigungsvorrichtung ist eingerichtet, um das Reaktivgas mit zumindest einem Teil des Reinigungsguts in Kontakt zu bringen, insbesondere innerhalb der Reinigungskammer und/oder innerhalb mindestens einer Desinfektionskammer.

Eine Idee der vorliegenden Erfindung besteht somit darin, mindestens eine Plasmaquelle zur Desinfektion zu verwenden. Insbesondere wurden in jüngerer Zeit Plasmaquellen entwickelt, welche einfach zu handhaben sind und Plasmen bei Raumtemperatur und/oder bei vergleichsweise niedrigen Temperaturen erzeugen können. Derartige Plasmen werden häufig auch als "kalte Plasmen" bezeichnet. Insbesondere kann ein kaltes Plasma Temperaturen von nicht mehr als 100°C aufweisen, insbesondere von nicht mehr als 80°C und beispielsweise von nicht mehr als 60°C oder sogar nicht mehr als 40°C. Unter einer Plasmaquelle ist dementsprechend im Rahmen der vorliegenden Erfindung eine Vorrichtung zu verstehen, welche eingerichtet ist, um mindestens ein Plasma zu zünden. Beispielsweise kann die Plasmaquelle, wie unten noch näher ausgeführt wird, zu diesem Zweck mindestens eine, vorzugsweise mindestens zwei Elektroden umfassen, beispielsweise wie unten ebenfalls noch näher ausgeführt wird, mindestens eine vollflächige Elektrode und mindestens eine Elektrode mit einer Mehrzahl von Öffnungen, beispielsweise eine Gitterelektrode. Auch andere Arten von Plasmaquellen sind jedoch grundsätzlich bekannt, beispielsweise Hochspannungsquellen, welche unter Verwendung einer Hochspannung ein Plasma erzeugen können. Bezüglich möglicher Plasmaquellen kann exemplarisch auf den oben genannten Stand der Technik verwiesen werden. Auch andere Plasmaquellen sind jedoch grundsätzlich einsetzbar.

Das mindestens eine Gas, in welchem das Plasma gezündet wird, kann ein speziell für die Zündung des Plasmas bereitgestelltes Gas sein, kann jedoch grundsätzlich insbesondere ein Sauerstoff enthaltendes Gas sein und vorzugsweise Luft. Dieses Gas kann in der Reinigungskammer vorhanden sein oder außerhalb der Reinigungskammer, wobei in letzterem Fall beispielsweise das erzeugte Reaktivgas in die Reinigungskammer überführt werden kann. Auch andere Ausgestaltungen sind jedoch grundsätzlich möglich. Insbesondere kann das Gas feuchte Luft sein, insbesondere Luft mit einer Luftfeuchtigkeit von mindestens 80%, vorzugsweise mindestens 90%, insbesondere mindestens 95% oder sogar mindestens 98% oder sogar 100%, beispielsweise in Form von Nebel. Anstelle oder zusätzlich einer Verwendung von trockener oder feuchter Luft als Gas, in welchem das Plasma gezündet wird, können auch andere Gase verwendet werden, oder der Luft können beispielsweise gezielt Komponenten zugesetzt werden, welche die Zündung des Plasmas und/oder die Zusammensetzung des Reaktivgases beeinflussen.

Unter einem Reaktivgas ist allgemein ein Gas zu verstehen, welches eine physikalische und/oder chemische und/oder biologische Wirkung, beispielsweise eine keimabtötende Wirkung aufweist. Das Reaktivgas ist gasförmig, kann jedoch grundsätzlich auch eine oder mehrere flüssige Komponenten umfassen, beispielsweise in Tröpfchenform oder auch als Aerosol. Das Reaktivgas kann insbesondere, wie unten noch näher ausgeführt wird, mindestens eine reaktive Komponente umfassen, vorzugsweise ausgewählt aus: Ozon, einer reaktiven Sauerstoffverbindung, einer reaktiven Stickstoffverbindung, atomarem Sauerstoff, atomarem Stickstoff, Wasserstoffperoxid. Auch andere Arten von Reaktivgasen sind jedoch grundsätzlich möglich. Alternativ oder zusätzlich kann das Reaktivgas, wie unten noch näher ausgeführt wird, mindestens ein Plasma umfassen, insbesondere das von der Plasmaquelle erzeugte Plasma und/oder einen Teil desselben, vorzugsweise mindestens ein kaltes Plasma gemäß der obigen Definition. Unter einem Plasma ist allgemein im Rahmen der vorliegenden Erfindung ein Gas zu verstehen, welches teilweise oder vollständig aus freien Ladungsträgern, insbesondere Ionen und/oder Elektronen, zusammengesetzt ist. Insbesondere kann das Plasma allgemein ein Gemisch aus neutralen und geladenen Teilchen, beispielsweise Atomen und/oder Molekülen, sein. Ein Verhältnis zwischen geladenen Teilchen und ungeladenen Teilchen in dem Plasma, beispielsweise ein Verhältnis zwischen geladenen Molekülen bzw. Ionen und ungeladenen Molekülen bzw. Ionen, kann beispielsweise von 10⁻⁸ - 10⁻¹¹ betragen, insbesondere 10⁻⁹ - 10⁻¹⁰.

Das Inkontaktbringen des Reaktivgases mit zumindest einem Teil des Reinigungsguts kann insbesondere derart erfolgen, dass mindestens eine Oberfläche des Reinigungsguts vollständig oder teilweise mit dem Reaktivgas in Kontakt gebracht wird, insbesondere überspült wird. Dieses Inkontaktbringen kann, wie oben ausgeführt, innerhalb der Reinigungskammer selbst erfolgen, in welcher auch die Beaufschlagung mit dem mindestens einen Reinigungsfluid erfolgt, und/oder innerhalb mindestens einer separaten Desinfektionskammer. Verschiedene Ausgestaltungen sind möglich. Das Plasma kann innerhalb der Reinigungskammer und/oder innerhalb der Desinfektionskammer selbst erzeugt werden oder in eine separaten Raum, beispielsweise einer separaten Plasmaquelle, wobei anschließend das durch das Plasma erzeugte Reaktivgas vorzugsweise in die Reinigungskammer und/oder die Desinfektionskammer eingebracht wird, beispielsweise durch Einsaugen und/oder Einblasen.

Ein Gedanke der vorliegenden Erfindung besteht also darin, eine desinfizierende Wirkung in der Reinigungsvorrichtung durch Verwendung eines durch ein Plasma erzeugten Reaktivgases herbeizuführen. Derartige Plasmaquellen und durch Plasmen erzeugte Reaktivgase sind aus dem Stand der Technik grundsätzlich bekannt. Beispielsweise werden in G. E. Morfill et al.: Nosocomial infections - A new approach towards preventive medicine using plasmas, New Journal of Physics 11 (2009) 115019 Plasmaquellen beschrieben, mittels derer sich sogenannte kalte Plasmen erzeugen lassen. Diese Plasmaquellen, welche auch im Rahmen der vorliegenden Erfindung einsetzbar sind, können beispielsweise eine oder mehrere Elektroden aufweisen, beispielsweise eine erste Elektrode, welche als vollflächige Elektrode, insbesondere als Kupferelektrode, ausgestaltet ist, und eine zweite Elektrode, die beispielsweise als Maschenelektrode ausgestaltet ist. Eine sinusförmige Wechselspannung kann an diese Elektroden angelegt werden, wobei ein Plasma gezündet wird. Dieses Plasma zeichnet sich in der Regel durch eine geringe Temperatur, beispielsweise Raumtemperatur, aus, und es bilden sich reaktive Gaskomponenten, insbesondere reaktive Sauerstoff- und Stickstoffverbindungen sowie atomarer Sauerstoff, Stickstoff und H₂O₂ durch Wechselwirkung mit Wasserdampf. Weiterhin wird in dieser Veröffentlichung sowie in M. Vogel: Hineinhalten statt waschen - Ein Plasmaspender vereinfacht die Desinfektion der Hände, Physik Journal, Januar 2010, Seite 16, eine desinfizierende Wirkung derartiger Plasmaquellen beschrieben. Erfindungsgemäß wird daher vorgeschlagen, eine oder mehrere Plasmaquellen, beispielsweise wie in dem genannten Stand der Technik beschrieben, auch in der Reinigungsvorrichtung gemäß der vorliegenden Erfindung einzusetzen.

Die Reinigungsvorrichtung kann insbesondere eingerichtet sein, um das Reaktivgas innerhalb der Reinigungskammer mit zumindest einem Teil des Reinigungsguts in Kontakt zu bringen. Alternativ oder zusätzlich kann das Inkontaktbringen jedoch auch außerhalb der Reinigungskammer erfolgen, insbesondere in einer separaten Desinfektionskammer, welche beispielsweise mit der Reinigungskammer in Verbindung stehen kann und/oder in einem gemeinsamen Gehäuse mit der Reinigungskammer angeordnet sein kann und/oder in einem verfahrensmäßigen Zusammenhang mit der Reinigungskammer stehen kann.

Die Reinigungsvorrichtung weist eine Einkammer-Reinigungsvorrichtung mit genau einer Reinigungskammer auf. Die Reinigungskammer kann zur Eingabe und/oder Entnahme des Reinigungsguts geöffnet und/oder verschlossen werden, beispielsweise durch mindestens eine Klappe und/oder mindestens einen Deckel und/oder mindestens einen Schieber und/oder mindestens eine Haube.

Die Reinigungsvorrichtung kann insbesondere mindestens eine Ansteuerung aufweisen. Die Ansteuerung kann insbesondere eingerichtet sein, um mindestens ein Reinigungsprogramm in der Reinigungsvorrichtung zu steuern. Beispielsweise kann die Ansteuerung eine zentrale oder eine dezentrale Ansteuerung sein. Die Ansteuerung kann beispielsweise mindestens eine Datenverarbeitungsvorrichtung und/oder mindestens eine Programmelektronik umfassen, um das mindestens eine Reinigungsprogramm zu steuern. In mindestens einem Reinigungsprogrammschritt kann das Reinigungsgut mit dem mindestens einen Reinigungsfluid beaufschlagt werden. In mindestens einem Desinfektionsschritt, welcher beispielsweise getrennt von dem mindestens einen Reinigungsprogrammschritt ausgestaltet sein kann, beispielsweise diesem nachgeschaltet sein kann, kann das Reinigungsgut mit dem Reaktivgas beaufschlagt werden, vorzugsweise nach Durchführung des Reinigungsprogrammschritts. Auf diese Weise können beispielsweise ein oder mehrere Reinigungsprogrammschritte durchgeführt werden, in welchen das Reinigungsgut mit dem Reinigungsfluid beaufschlagt wird, beispielsweise mit unterschiedlichen Reinheitsgraden und/oder unterschiedlichen Zusammensetzungen des Reinigungsfluids. Anschließend kann beispielsweise der mindestens eine Desinfektionsschritt durchgeführt werden. Auch eine andere Reihenfolge der Programmschritte ist grundsätzlich denkbar, beispielsweise indem mindestens ein Desinfektionsschritt vor mindestens einem Reinigungsprogrammschritt durchgeführt wird. Zudem kann das Reinigungsprogramm einen oder mehrere weitere Programmschritte umfassen, beispielsweise einen oder mehrere Trockungsschritte. Der mindestens eine Desinfektionsschritt kann vor, während oder nach dem Trocknungsschritt durchgeführt werden.

Die Einkammer-Reinigungsvorrichtung weist mindestens ein Reinigungs- und Desinfektionsgerät zur Reinigung von Pflegeutensilien auf, insbesondere zur Reinigung von Steckbecken, Bettpfannen, Urinflaschen, Waschschüsseln oder ähnlichen Pflegeutensilien, welche zur Aufnahme flüssiger und/oder fester menschlicher Ausscheidungen von mindestens 100 ml, vorzugsweise mindestens 500 ml oder sogar mindestens 1000 ml geeignet sind. Unter einem Reinigungs- und Desinfektionsgerät ist allgemein eine Vorrichtung zu verstehen, welche eine Fluidreinigung und eine Desinfektion des Reinigungsguts durchführen kann. Das Reinigungs- und Desinfektionsgerät umfasst mindestens einen Abfluss zur Entsorgung von in den Pflegeutensilien enthaltenen Ausscheidungen, beispielsweise mit Flüssigkeitsmengen von mindestens 100 ml, vorzugsweise mindestens 500 ml oder sogar mindestens 1000 ml. Beispielsweise kann ein Abfluss mit einem Durchmesser oder Äquivalentdurchmesser von mindestens 30 mm, vorzugsweise mindestens 50 mm, besonders bevorzugt mindestens 75 mm oder sogar mindestens 100 mm vorgesehen sein. Der Abfluss ist mit einem Geruchsverschluss, vorzugsweise mindestens einem Siphonbogen, ausgestaltet. In diesem Geruchsverschluss kann insbesondere mindestens eine Flüssigkeitsmenge als Geruchsverschluss vorgesehen sein. Die Reinigungsvorrichtung ist eingerichtet, um das Reaktivgas, vorzugsweise nach Beaufschlagung des Reinigungsguts, aus der Reinigungskammer in den Abfluss hinter den Geruchsverschluss abzuführen, insbesondere durch eine Zwangsverdrängung aus der Reinigungskammer, beispielsweise durch Dampf und/oder Druckluft und/oder ein anderes Druckgas, insbesondere in den Abfluss hinter einem Siphonbogen. So kann das Abführen des Reaktivgases aus der Reinigungskammer beispielsweise unter Umgehung eines Siphonbogens erfolgen.

Alternativ oder zusätzlich zu den oben beschriebenen Ausgestaltungen kann die Reinigungsvorrichtung auch mindestens ein Transportsystem aufweisen, wobei das Transportsystem eingerichtet sein kann, um das Reinigungsgut durch die Reinigungskammer oder einen Teil derselben hindurch zu transportieren. Das Transportsystem kann beispielsweise ein Band, eine Gliederkette, einen Rollenantrieb oder andere Arten bekannter Transportsysteme umfassen. Der Transport des Reinigungsguts durch die mindestens eine Reinigungskammer hindurch kann beispielsweise mit gleichmäßiger Geschwindigkeit oder auch mit variierender Geschwindigkeit erfolgen, wobei beispielsweise eine Transportgeschwindigkeit eine Intensität und/oder Dauer einer Beaufschlagung mit dem Reinigungsfluid bestimmen kann.

Die Reinigungskammer kann eine Zone oder auch insbesondere mehrere Zonen umfassen. Beispielsweise können diese Zonen in einem gemeinsamen Gehäuse angeordnet sein. Diese Zonen können insbesondere mindestens eine Reinigungszone umfassen, insbesondere eine Reinigungszone ausgewählt aus einer Vorabräumzone, einer Hauptreinigungszone, einer Pumpenklarspülzone und einer Frischwasserklarspülzone. Auch beliebige Kombinationen der genannten Reinigungszonen sind denkbar. Beispielsweise kann sich an eine Vorabräumzone in einer Transportrichtung des Transportsystems mindestens eine Hauptreinigungszone anschließen, gefolgt vorzugsweise von mindestens einer Klarspülzone, beispielsweise mindestens einer Pumpenklarspülzone und anschließend mindestens einer Frischwasserklarspülzone. Weiterhin kann vorzugsweise, insbesondere in Transportrichtung der mindestens einen Reinigungszone nachgeordnet, mindestens eine Trocknungszone vorgesehen sein, beispielsweise mindestens eine Trocknungszone mit mindestens einem Trocknungsgebläse, über welches das Reinigungsgut mit beispielsweise Warmluft zur Trocknung beaufschlagt werden kann. Weiterhin kann mindestens eine Desinfektionszone in der Reinigungsvorrichtung vorgesehen sein, beispielsweise innerhalb der Reinigungskammer und/oder in mindestens einer separaten Desinfektionskammer. Die Desinfektionszone kann insbesondere derart ausgestaltet sein, dass mittels des Transportsystems das Reinigungsgut durch die mindestens eine Desinfektionszone transportiert wird. Die mindestens eine Desinfektionszone kann auch vollständig oder teilweise mit mindestens einer der vorgenannten Zonen zusammengefasst sein, beispielsweise einer oder mehreren der Reinigungszonen und/oder der mindestens einen Trocknungszone. Insbesondere kann die Desinfektionszone vollständig oder teilweise in die mindestens eine Trocknungszone integriert sein. Vorzugsweise erfolgt nach Durchlaufen der mindestens einen Desinfektionszone keine Beaufschlagung mit Reinigungsfluid mehr, sodass vorzugsweise die mindestens eine Desinfektionszone zumindest weitgehend an ein Ende der Reinigungsvorrichtung angeordnet ist. In der Desinfektionszone kann allgemein mindestens eine Beaufschlagung des Reinigungsguts mit dem mindestens einen Reaktivgas erfolgen.

Wie oben ausgeführt, wird mit dem mindestens einen Reaktivgas zumindest ein Teil des Reinigungsguts beaufschlagt, indem das Reinigungsgut vollständig oder teilweise mit dem Reaktivgas in Kontakt gebracht wird. Weiterhin kann das Reaktivgas auch eingesetzt werden, um Bestandteile der Reinigungsvorrichtung zu reinigen und/oder zu desinfizieren. Insbesondere kann die Reinigungsvorrichtung weiterhin eingerichtet sein, um zumindest einen Teil des Transportsystems mit dem Reaktivgas in Kontakt zu bringen, insbesondere mindestens ein Transportband und/oder mindestens einen Transportkorb.

Die Reinigungsvorrichtung kann weiterhin eingerichtet sein, um innerhalb der Reinigungskammer einen Gasstrom entgegen einer Transportrichtung des Transportsystems zu erzeugen. Beispielsweise kann der Gasstrom durch mindestens ein Gebläse und/oder durch mindestens eine Absaugvorrichtung erzeugt werden. Beispielsweise kann in der mindestens einen optionalen Trocknungszone mindestens ein Gebläse vorgesehen sein, welches den Gasstrom vollständig oder teilweise erzeugt, beispielsweise indem dieser Gasstrom durch entsprechende Leitelemente, beispielsweise Leitbleche, entsprechend gerichtet wird. Alternativ oder zusätzlich kann beispielsweise in einem Einlaufbereich der Reinigungsvorrichtung mindestens eine Absaugung vorgesehen sein. Die Reinigungsvorrichtung kann insbesondere an einem Einlaufbereich mindestens eine Absaugung umfassen, mittels derer der Gasstrom entgegen der Transportrichtung des Transportsystems vollständig oder teilweise erzeugt werden kann, gegebenenfalls in Zusammenwirkung mit mindestens einem Gebläse. Die Reinigungsvorrichtung kann insbesondere an einem Einlaufbereich mindestens eine Absaugung mit mindestens einer Aufbereitungsvorrichtung umfassen, wobei die Aufbereitungsvorrichtung eingerichtet sein kann, um das Reaktivgas zumindest teilweise aus einer abgesaugten Luft zu entfernen. Die mindestens eine Aufbereitungsvorrichtung kann beispielsweise mindestens einen Filter und/oder mindestens einen Katalysator umfassen, mittels derer beispielsweise das Reaktivgas und/oder Teile des Reaktivgases aus der abgesaugten Luft entfernt werden und/oder in andere chemische Verbindungen umgewandelt werden, beispielsweise weniger reaktive chemische Verbindungen.

Allgemein ist, unabhängig von der Ausgestaltung der Reinigungsvorrichtung, die Reinigungsvorrichtung eingerichtet, um das Reaktivgas nach Kontakt mit zumindest einem Teil des Reinigungsguts und optional mit zumindest einem Teil der Reinigungsvorrichtung durch mindestens eine Aufbereitungsvorrichtung zu leiten, insbesondere mindestens einen Filter und/oder mindestens einen Katalysator. Auf diese Weise können beispielsweise schädliche Bestandteile aus einem Abgas der Entsorgungsvorrichtung zumindest teilweise entfernt werden, beispielsweise Ozon, Wasserstoffperoxid oder andere reaktive Komponenten. Beispielsweise kann mindestens ein Aktivkohlefilter vorgesehen sein. Alternativ oder zusätzlich kann mindestens ein Katalysator vorgesehen sein, beispielsweise mindestens ein Platin-Katalysator und/oder mindestens ein Palladium-Katalysator.

Die Reinigungsvorrichtung kann allgemein mindestens eine Druckvorrichtung aufweisen, wobei die Druckvorrichtung eingerichtet sein kann, um mindestens einen Überdruck und/oder mindestens einen Unterdruck in mindestens einem Teil der Reinigungsvorrichtung, beispielsweise der mindestens einen Reinigungskammer und/oder der mindestens einen Desinfektionskammer, zu erzeugen. Beispielsweise kann die Druckvorrichtung mindestens eine Saugvorrichtung und/oder mindestens ein Gebläse aufweisen. Die Druckvorrichtung kann insbesondere eingerichtet sein, um das Reaktivgas nach Kontakt mit zumindest einem Teil der Reinigungsvorrichtung in ein Abflusssystem abzuleiten. Dieses Ableiten kann beispielsweise durch Absaugen, Verdrängen, Auswaschen oder andere Arten der Entfernung des Reaktivgases, beispielsweise aus der Reinigungskammer und/oder der Desinfektionskammer, erfolgen.

Weitere mögliche Ausgestaltungen der Erfindung betreffen die Ausgestaltung der mindestens einen Plasmaquelle. Allgemein können beispielsweise die in dem oben genannten Stand der Technik beschriebenen Plasmaquellen zum Einsatz kommen und/oder auch andere Plasmaquellen. Die Plasmaquelle kann, wie oben bereits ausgeführt, insbesondere mindestens eine erste Elektrode und mindestens eine zweite Elektrode aufweisen. Diese Elektroden können beispielsweise parallel zueinander angeordnet sein. Beispielsweise können die Elektroden als flächige Elektroden ausgestaltet sein, mit einer Elektrodenfläche von mindestens 100 mm², vorzugsweise mindestens 1000 mm², insbesondere mindestens 10 000 mm² oder sogar mindestens 100 000 mm² oder mindestens 1 000 000 mm². Weist die Plasmaquelle mindestens zwei Elektroden auf, so kann mindestens eine der Elektroden eine Mehrzahl von Öffnungen aufweisen, beispielsweise eine Mehrzahl von Perforationen und/oder Maschen. Insbesondere kann mindestens eine der Elektroden als Gitterelektrode ausgestaltet sein, beispielsweise in Form eines Drahtgitters. Als Elektrodenmaterialien kommen insbesondere metallische Materialien in Betracht. Beispielsweise kann mindestens eine der Elektroden als Metallblech ausgestaltet sein, beispielsweise als Kupferblechelektrode. Die Elektrode mit der Mehrzahl der Öffnungen kann beispielsweise als Maschenelektrode aus Kupferdraht und/oder rostfreiem Stahl ausgestaltet sein. Mindestens eine der Elektroden, beispielsweise die Elektrode mit der Mehrzahl der Öffnungen, kann geerdet sein.

Zwischen den mindestens zwei Elektroden kann insbesondere mindestens ein Dielektrikum angeordnet sein. Beispielsweise kann dieses Dielektrikum einen Kunststoff mit isolierenden Eigenschaften und/oder ein keramisches Material umfassen. Das Dielektrikum kann beispielsweise eine relative Permittivität εᵣ von 1-5 aufweisen, beispielsweise von 1,1-4, insbesondere von 1,5-2,5 und besonders bevorzugt 2. Als Kunststoffmaterial kommt beispielsweise mindestens ein Polymer in Betracht, beispielsweise Polytetrafluorethylen. Alternativ oder zusätzlich sind jedoch auch andere dielektrische Materialien einsetzbar. Diese beschriebene Anordnung kann auch als Elektrodenbaugruppe beschrieben werden. Eine einzelne solche Elektrodenbaugruppe kann zusammen mit einer elektrischen Ansteuerung das gewünschte Plasma erzeugen. Es können auch mehrere Elektrodenbaugruppen in räumlicher Nähe angeordnet sein, um ein größeres Volumen mit Plasma anzureichern. Mindestens zwei dieser Elektrodenbaugruppen können beispielsweise in einem Abstand von 0,5 mm - 50 mm voneinander angeordnet sein, insbesondere von 1 mm - 40 mm.

Die Plasmaquelle kann insbesondere auf eine oder mehrere der folgenden Arten ausgestaltet sein. So kann die Plasmaquelle insbesondere innerhalb der Reinigungskammer angeordnet sein und eingerichtet sein, das Plasma innerhalb der Reinigungskammer zu zünden. In diesem Fall steht das Gas, in welchem das Plasma gezündet wird, insbesondere unmittelbar mit der in der Reinigungskammer herrschenden Atmosphäre in Verbindung oder ist zumindest teilweise identisch mit derselben. Alternativ oder zusätzlich kann die Plasmaquelle jedoch auch außerhalb der Reinigungskammer angeordnet sein, und die Reinigungsvorrichtung kann eingerichtet sein, um das Reaktivgas in zumindest einen Teil der Reinigungskammer zu leiten, insbesondere durch ein Gebläse, eine Saugvorrichtung oder eine Überdruckvorrichtung. In diesem Fall kann die externe Plasmaquelle beispielsweise als Plasmaspender eingerichtet sein, und das durch die Plasmaquelle erzeugte Reaktivgas kann in die Reinigungskammer transferiert werden, beispielsweise durch Einsaugen und/oder Einblasen. Beispielsweise kann der externe Plasmaspender in Form eines Anbaus und/oder eines Stutzens an der Reinigungskammer angeordnet sein, und das erzeugte Reaktivgas kann durch diesen Stutzen in die Reinigungskammer eingeleitet werden. Der Stutzen und/oder Anbau kann beispielsweise über ein Gebläse verfügen, mittels dessen das Reaktivgas in die Reinigungskammer eingeleitet wird.

Die Plasmaquelle kann weiterhin insbesondere mindestens eine elektrische Energiequelle aufweisen. Diese elektrische Energiequelle kann eingerichtet sein, um die Elektroden mit einer Wechselspannung zu beaufschlagen, worunter die Möglichkeit der Ausgestaltung als Wechselspannungsquelle und/oder als Wechselstromquelle subsummiert werden kann. Die elektrische Energiequelle kann beispielsweise eine Spannung in einem Bereich von 1 kV-100 kV bereitstellen, beispielsweise eine Spitze-zu-Spitze-Spannung, vorzugsweise eine Spannung von 5 kV-50kV, besonders bevorzugt von 10 kV-25 kV und insbesondere 18 kV. Ist eine Wechselspannungsquelle vorgesehen, so kann diese beispielsweise eine Frequenz im Bereich von 100 Hz-100 kHz aufweisen, insbesondere im Bereich von 1 kHz-50kHz, vorzugsweise von 5 kHz-20 kHz und besonders bevorzugt bei ca. 12,5 kHz.

Wie oben beschrieben, kann das Gas, in welchem das Plasma gezündet wird, insbesondere Sauerstoff umfassen, beispielsweise mit einem Anteil von 1 % - 80 %, insbesondere von 10 % - 30 % und besonders bevorzugt von ca. 20 %. Insbesondere kann das Gas Luft sein. Die Luft kann vorzugsweise einen hohen Feuchtegehalt aufweisen, beispielsweise eine relative Luftfeuchtigkeit von mehr als 90 %, vorzugsweise von mehr als 95 %. Alternativ oder zusätzlich zur Verwendung von Luft können jedoch auch andere Gase verwendet werden und/oder es können der Luft gezielt Komponenten beigesetzt werden, welche die Zündung und/oder Zusammensetzung des Plasmas und/oder die Zusammensetzung des Reaktivgases gezielt beeinflussen.

Das Reaktivgas kann, wie oben ausgeführt, insbesondere mindestens ein Plasma umfassen, insbesondere ein kaltes Plasma mit einer Temperatur von weniger als 100 °C, vorzugsweise von weniger als 80 °C und besonders bevorzugt von weniger als 60 °C oder sogar weniger als 40 °C. Entsprechende Temperaturen können auch für das Reaktivgas gelten. Das Plasma kann insbesondere ein Verhältnis geladener Teilchen zu ungeladenen Teilchen von 10⁻⁸ - 10⁻¹¹ aufweisen, vorzugsweise von 10⁻⁹ - 10⁻¹⁰. Das Reaktivgas kann insbesondere einen Druck aufweisen, welcher nicht erheblich von einem Normaldruck abweicht, beispielsweise einen Druck von 100 mbar - 2000 mbar, insbesondere einen Druck von 500 mbar - 1500 mbar, vorzugsweise einen Druck von 800 mbar - 1200 mbar und besonders bevorzugt Normaldruck. Das Reaktivgas kann insbesondere mindestens eine durch ein durch das Plasma erzeugbare Verbindung aufweisen, insbesondere eine gasförmige Verbindung, wobei jedoch grundsätzlich auch flüssige Verbindungen in dem Reaktivgas enthalten sein können, neben gasförmigen Verbindungen. Insbesondere kann das Reaktivgas mindestens eine durch das Plasma erzeugbare Verbindung aufweisen, ausgewählt aus der Gruppe bestehend aus: Ozon, atomarem Sauerstoff, atomarem Stickstoff, Wasserstoffperoxid.

Die Plasmaquelle kann, wie oben ausgeführt, insbesondere weiterhin mindestens ein Gebläse aufweisen, wobei das Gebläse eingerichtet ist, um das Reaktivgas in zumindest einen Teil der Reinigungsvorrichtung zu befördern, insbesondere in mindestens eine Reinigungskammer. Beispielsweise kann das Gebläse derart eingerichtet sein, dass das Reaktivgas unmittelbar auf das Reinigungsgut geleitet wird. Bei der Reinigung anderer Arten von Reinigungsgut kann die Richtung des Reaktivgases auf andere Weise gewählt werden. Das Gebläse kann beispielsweise mit einer Düse verbunden sein, sodass eine Strahlrichtung des Reaktivgases gezielt gewählt werden kann. Beispielsweise kann das Reaktivgas auch in einen Innenraum von Steckbecken, Urinflaschen, oder anderen Gefäßen zur Aufnahme menschlicher Ausscheidungen gerichtet werden. Bei der Reinigung persönlicher Schutzausrüstung kann ebenfalls eine Richtung des Reaktivgases gezielt gewählt werden, beispielsweise in einen Innenbereich von Masken, auf ein Mundstück, in einen Innenbereich von Schläuchen oder gezielt auf Bereiche, welche mit Schweiß, Speichel, anderen Arten von Körperflüssigkeiten oder anderen Arten von Verunreinigungen in Berührung kommen können.

In einem weiteren Aspekt der vorliegenden Erfindung wird ein Verfahren zur Reinigung von Reinigungsgut vorgeschlagen. Dieses Verfahren wird unter Verwendung einer Reinigungsvorrichtung gemäß einer oder mehreren der oben beschriebenen Ausgestaltungen durchgeführt, sodass für mögliche Ausgestaltungen des Verfahrens auf die möglichen Ausgestaltungen der Reinigungsvorrichtung verwiesen werden kann. Auch andere Ausgestaltungen sind jedoch grundsätzlich möglich. Bei dem Verfahren wird das Reinigungsgut in mindestens einer Reinigungskammer mit mindestens einem Reinigungsfluid beaufschlagt. Weiterhin wird mittels mindestens einer Plasmaquelle ein Plasma in mindestens einem Gas gezündet und mindestens ein Reaktivgas erzeugt. Das Reaktivgas wird mit zumindest einem Teil des Reinigungsguts in Kontakt gebracht. Dieses Inkontaktbringen des Reaktivgases mit dem Reinigungsgut kann insbesondere innerhalb der Reinigungskammer und/oder innerhalb mindestens einer Desinfektionskammer erfolgen.

Die vorgeschlagene Reinigungsvorrichtung und das vorgeschlagene Verfahren weisen gegenüber bekannten Vorrichtungen und Verfahren eine Vielzahl von Vorteilen auf. Insbesondere lässt sich eine Reinigung und/oder Desinfektion mittels der Plasmaquelle und des durch diese erzeugten Reaktivgases, insbesondere mit dem mindestens einen Plasma, auf einfache, zuverlässige und vergleichsweise kostengünstige Art realisieren. So kann die Desinfektion dadurch erfolgen, dass die Reinigungskammer, beispielsweise ein Behandlungsraum und/oder eine Waschkammer, mit dem Reaktivgas, insbesondere mit dem mindestens einen Plasma, gefüllt wird. Beispielsweise können in einer Wand und/oder an mehreren Wänden der Reinigungskammer und/oder des Behandlungsraums, beispielsweise der Desinfektionskammer, Elektroden eingebracht werden, die beispielsweise unter Einsatz von elektrischer Energie das Gas in der Reinigungskammer bzw. Desinfektionskammer, insbesondere normale Luft, zumindest teilweise in ein Plasma umwandeln können. Dieser Behandlungsschritt kann insbesondere bei Normaldruck erfolgen oder unter davon leicht abweichenden Bedingungen, beispielsweise Überdruck oder Unterdruck. Alternativ oder zusätzlich kann das Plasma auch in einem separaten Plasmaspender erzeugt werden, der mit der Reinigungskammer und/oder Desinfektionskammer (beide Möglichkeiten werden im Folgenden auch allgemein als "Behandlungsraum" bezeichnet), beispielsweise durch eine Rohrleitung verbunden ist. Während eines Desinfektionsschritts kann das Plasma beispielsweise durch ein Gebläse in den Behandlungsraum eingeblasen werden.

Die keimabtötende Wirkung von Plasmen, insbesondere kalter Plasmen, und/oder die keimabtötende Wirkung von durch derartige Plasmen erzeugten Reaktivgasen, ist beispielsweise aus dem oben beschriebenen Stand der Technik bekannt. Erfindungsgemäß wird diese keimabtötende Wirkung auf das Aufbereiten von Utensilien aus dem Bereich der Patientenpflege übertragen.

Die Erzeugung und/oder Einleitung des Reaktivgases, insbesondere mit dem mindestens einen Plasma, kann beispielsweise in einem relativ trockenen Behandlungsraum erfolgen und/oder in einem Behandlungsraum, der mit feuchter Atmosphäre gefüllt ist. Es ist auch vorstellbar, dass beispielsweise während der Plasmaerzeugung und/oder Plasmaeinleitung zusätzliche Behandlungsflüssigkeit und/oder mindestens eine Gaskomponente in den Behandlungsraum eingesprüht wird, um die Zusammensetzung und/oder die Wirkung des Reaktivgases bzw. des Plasmas zu beeinflussen.

In einer Reinigungsvorrichtung, in der das zu behandelnde Reinigungsgut während der gesamten Behandlung stationär in einer Reinigungskammer verbleibt, kann die Behandlung beispielsweise wie oben beschrieben, in Form einer Abfolge von Programmschritten ablaufen. Derartige Programme sind bei den oben beschriebenen Reinigungs- und Desinfektionsgeräten in vielen Fällen einsetzbar. In einem derartigen Reinigungsprogramm mit einer Mehrzahl von Programmschritten kann beispielsweise mindestens ein Schritt erfolgen, in welchem das Reinigungsgut mit dem Reaktivgas, insbesondere enthaltend das mindestens eine Plasma, behandelt wird. Dieser Behandlungsschritt, welcher im Folgenden auch als Plasmaschritt bezeichnet wird, kann beispielsweise als letzter Schritt vor einem Programmende erfolgen. Alternativ oder zusätzlich ist es jedoch auch denkbar, dass der Plasmaschritt schon beispielsweise vor einer letzten Klarspülung erfolgt. Dadurch können eventuell im Plasma entstehende unerwünschte Stoffe ausgewaschen und damit beseitigt werden. Alternativ oder zusätzlich ist es weiterhin vorstellbar, dass der Bedarf an Klarspülmittel, welcher beispielsweise in üblichen Reinigungs- und Desinfektionsgeräten besteht, durch den Einsatz des Plasmas reduziert werden kann, je nach Gegebenheiten sogar bis auf Null. Weiterhin ist es vorstellbar, dass schon in früheren Phasen der Behandlung mindestens ein Plasmaschritt eingefügt wird, beispielsweise um eine Reinigungswirkung zu verbessern. Die Anwendung mindestens eines Plasmaschritts kann unabhängig davon erfolgen, ob während eines Programmablaufs das Reinigungsfluid in einem Tank ausgetauscht wird oder nicht.

Wenn der Plasmaschritt als letzter Programmschritt erfolgt, so kann das Reaktivgas, insbesondere das Plasma, beispielsweise mit einem Gebläse in den Behandlungsraum eingebracht werden und dadurch beispielsweise auch eine Trocknungswirkung am Reinigungsgut erreicht werden. Vorteilhaft dabei ist insbesondere, dass automatisch die angesaugte Trocknungsluft desinfiziert werden kann, während diese beispielsweise den Plasmaspender durchströmt. Beispielsweise kann zu diesem Zweck, wie oben ausgeführt, eine Plasmaquelle mit einem Gebläse vorgesehen sein. Beispielsweise kann die Reinigungsvorrichtung einen Ansaugstutzen umfassen, welcher in der Plasmaquelle mündet und/oder innerhalb dessen die Plasmaquelle angeordnet ist. So kann beispielsweise das Plasma zumindest teilweise in angesaugter Frischluft erzeugt werden, sodass diese in das Reaktivgas umgewandelt wird und/oder das Reaktivgas enthält, bevor diese angesaugte Frischluft dann beispielsweise in den Behandlungsraum, insbesondere die Reinigungskammer und/oder die Desinfektionskammer, eingebracht wird. Beispielsweise kann die Reinigungsvorrichtung derart ausgestaltet sein, dass automatisch die gesamte angesaugte Frischluft und/oder Trocknungsluft durch die Plasmaquelle desinfiziert wird.

Wie oben beschrieben, kann das Plasma beispielsweise unmittelbar in der Reinigungskammer und/oder der Desinfektionskammer gezündet werden. Alternativ oder zusätzlich ist es jedoch auch vorstellbar, mindestens eine Plasmaquelle, welche als Plasmaspender ausgestaltet sein kann, derart auszugestalten, dass das von dieser Plasmaquelle erzeugte Reaktivgas, insbesondere mit dem mindestens einen Plasma, mindestens einen optional vorhandenen Tank für das Reinigungsfluid und/oder einzelne oder alle Leitungen eines optionalen Rohrleitungssystems durchströmt, sodass diese vollständig oder teilweise mit dem Reaktivgas angefüllt werden. Dadurch lässt sich eine vollständige oder teilweise Systemdesinfektion der Reinigungsvorrichtung erreichen. Besonders bevorzugt ist es daher, wenn die Plasmaquelle derart ausgestaltet ist, dass das Reaktivgas mit zumindest einem Teile der Fluidquelle in Kontakt gebracht wird. Beispielsweise kann das Reaktivgas mit einer oder mehreren der folgenden Komponenten der Fluidquelle in Kontakt gebracht werden: mindestens einem Fluidtank, beispielsweise einem Waschtank und/oder einem Nachspültank; mindestens einer Rohrleitung eines Rohrleitungssystems der Fluidquelle, beispielsweise einer Frischwasserleitung und/oder einer Waschleitung und/oder einer Nachspülleitung; mindestens einer Düse der Fluidquelle. Insbesondere kann die Plasmaquelle derart ausgestaltet sein, dass das Reaktivgas in mindestens einem Fluidtank der Reinigungsvorrichtung erzeugt wird. Beispielsweise kann das Plasma in einem Gasvolumen, beispielsweise einem Luftvolumen, oberhalb eines Flüssigkeitsspiegels in dem Fluidtank gezündet werden. Beispielsweise kann ein Plasma in einem Wassertank des Reinigungs- und Desinfektionsgeräts erzeugt werden. Das Reaktivgas kann beispielsweise auch mit anderen Bestandteilen der Reinigungsvorrichtung in Kontakt kommen. So können beispielsweise neben dem Reinigungsfluid, insbesondere einem wässrigen Reinigungsfluid, sowie einer oder mehreren Leitungen, welche das Reinigungsfluid führen, gegebenenfalls auch vorhandene Luftkanäle und/oder darin befindliche Einbauten, wie beispielsweise mindestens ein Wärmetauscher, mit dem Reaktivgas in Kontakt gebracht werden, sodass sich auch diese, alternativ oder zusätzlich zu den oben genannten Komponenten, desinfizieren lassen.

Eine Plasmabehandlung zur Keimreduktion und/oder Desinfektion kann alleine oder in Kombination mit chemischen Desinfektionsverfahren eingesetzt werden oder auch alleine oder in Kombination mit thermischen Desinfektionsverfahren. Dementsprechend kann die Reinigungsvorrichtung optional zusätzlich zu der mindestens einen Plasmaquelle weiterhin mindestens eine Dosiervorrichtung zur Einbringung mindestens eines chemischen Desinfektionsmittels und/oder mindestens eine thermische Desinfektionsvorrichtung umfassen, beispielsweise mindestens eine Dampfquelle. Besonders bevorzugt ist es jedoch, wenn die Reinigungsvorrichtung ohne eine chemische Desinfektionsvorrichtung und/oder ohne eine thermische Desinfektionsvorrichtung ausgestaltet ist und eine Desinfektion allein aufgrund des Reaktivgases erfolgt.

Im Gegensatz zu herkömmlichen Verfahren und Vorrichtungen zur Desinfektion von Reinigungsgut erfordert die Erzeugung eines Plasmas und daraus eines Reaktivgases einen vergleichsweise geringen Energieeinsatz. Hierdurch unterscheidet sich die Erfindung beispielsweise deutlich von Reinigungsvorrichtungen, welche ausschließlich auf einer thermischen Desinfektion basieren, beispielsweise ausschließlich auf einer Dampferzeugung. So kann beispielsweise auf eine thermische Desinfektion vollständig verzichtet werden, oder es kann eine thermische Desinfektion gegenüber herkömmlichen Vorrichtungen und Verfahren deutlich reduziert werden, sodass der gesamte Energiebedarf der Reinigungsvorrichtung deutlich reduziert werden kann.

Weiterhin kann eine Einwirkdauer des Reaktivgases auf die zu desinfizierenden Bestandteile, insbesondere das Reinigungsgut, deutlich niedriger ausgestaltet sein als beispielsweise eine Einwirkdauer von Hitze und/oder chemischen Desinfektionsmitteln. Hierdurch ergeben sich Vorteile hinsichtlich der Dauer des gesamten Prozesses. In Programmautomaten kann beispielsweise die Gesamtdauer des Reinigungsprogramms deutlich gegenüber herkömmlichen Programmautomaten verkürzt werden.

Weiterhin ergeben sich Vorteile hinsichtlich der bei einer Desinfektion herrschenden Temperaturen. Beispielsweise kann das Reinigungsgut insgesamt während der gesamten Behandlung deutlich geringeren Maximaltemperaturen ausgesetzt sein. Beispielsweise kann die Behandlung in der Reinigungsvorrichtung bzw. mittels des vorgeschlagenen Verfahrens derart erfolgen, dass das Reinigungsgut auf Temperaturen von weniger als 95°C, vorzugsweise von weniger als 90°C und besonders bevorzugt von weniger als 85°C oder sogar weniger als 80°C aufgeheizt wird. Es sind sogar Verfahren und Vorrichtungen realisierbar, bei denen eine Aufheizung auf nicht mehr als 70°C, nicht mehr als 60°C oder sogar nicht mehr als 50°C erfolgt. Dementsprechend kann das Reinigungsgut am Ende der Behandlung, beispielsweise nach Ablauf des Reinigungsprogramms, schnell wieder entnommen und wieder verwendet werden. Eine Gefahr einer Verletzung eines Bedienpersonals durch heißes Reinigungsgut kann deutlich reduziert oder sogar vollständig ausgeschlossen werden. Weiterhin kann aufgrund der Möglichkeit der Realisierung niedriger Temperaturen auch Reinigungsgut desinfiziert werden, welches gegenüber höheren Temperaturen empfindlich ist. Beispielsweise können Kunststoffe desinfiziert werden, welche eine vergleichsweise geringe thermische Beständigkeit aufweisen, beispielsweise eine geringe Wärmeformbeständigkeit, beispielsweise eine Wärmeformbeständigkeitstemperatur HDT von weniger als 90°C, insbesondere von weniger als 85°C.

Als weiterer Vorteil ist zu verzeichnen, dass auf den Einsatz gefährlicher Stoffe verzichtet werden kann oder dass ein Einsatz derartiger gefährlicher und/oder umweltgefährdender Stoffe zumindest weitgehend vermieden oder vermindert werden kann. Beispielsweise kann auf den Einsatz chemischer Desinfektionsmittel verzichtet werden, oder die Menge derartiger chemischer Desinfektionsmittel, welche zum Einsatz kommen, kann zumindest reduziert werden. Hierdurch steigen die Umweltverträglichkeit und die Benutzerfreundlichkeit.

Weiterhin können durch die Einwirkung von Reaktivgasen und insbesondere von Plasma auch die Eigenschaften von Oberflächen verändert werden. Beispielsweise können die Oberflächeneigenschaften des Reinigungsguts und/oder von Bestandteilen der Reinigungsvorrichtung hinsichtlich ihrer Benetzbarkeit verändert werden. Derartige Effekte sind beispielsweise aus der Oberflächenbehandlung von Kunststoffteilen, beispielsweise vor einer Lackierung, bekannt. So ist es beispielsweise denkbar, dass sich durch die Einwirkung des mindestens einen Reaktivgases, insbesondere des Plasmas, ein positiver Nebeneffekt hinsichtlich eines Bedarfs und/oder Verbrauchs von Klarspüler einstellt.

Weiterhin ist als Vorteil der Einwirkung des Reaktivgases, insbesondere des Plasmas, zu verzeichnen, dass eine Desinfektionswirkung auch in Bereichen erzielt wird, welche mittels üblicher Desinfektionsverfahren in vielen Fällen nicht erreicht werden. So kann beispielsweise das mindestens eine Reaktivgas, insbesondere das mindestens eine Plasma, auch in Hohlräume eindringen, beispielsweise in Hohlräume von Pflegeutensilien wie beispielsweise Steckbecken, Urinflaschen oder ähnlichen Pflegeutensilien. Beispielsweise kann ein Plasma in einem weiten Bereich gebildet werden, beispielsweise überall dort, wo ein elektrisches Feld herrscht, und eine Plasmabildung kann somit über einen weiten Bereich innerhalb der Reaktionskammer erfolgen. Alternativ oder zusätzlich kann das Reaktivgas nach der Bildung mittels des mindestens einen Plasmas sich über einen weiten Bereich der Reinigungskammer und/oder der Desinfektionskammer erstrecken, beispielswese indem das Plasma aktiv in diesen Bereich transferiert wird und/oder beispielsweise durch Diffusionsprozesse. Auf diese Weise lässt sich eine extensive Desinfektionswirkung erzielen. Durch das mindestens eine Reaktivgas, insbesondere das mindestens eine Plasma, lassen sich, wie oben beschrieben, auch beispielsweise Vorratstanks, Flüssigkeitsleitungen, Luftkanäle, Wärmetauscher und andere Komponenten der Reinigungsvorrichtung desinfizieren. Hierdurch wird eine höhere Funktionssicherheit der Reinigungsvorrichtung erzielt, und es lassen sich beispielsweise auch Geruchsbelästigungen in der Umgebung der Reinigungsvorrichtung verhindern oder zumindest vermindern. Insbesondere lassen sich, neben dem Reinigungsgut, gegebenenfalls auch weitere Bestandteile der Reinigungsvorrichtung, einschließlich beispielsweise des Leitungssystems und/oder von Einbauten der Reinigungsvorrichtung, desinfizieren.

Die Plasmaquelle lässt sich auf verschiedene Weise in der Reinigungsvorrichtung integrieren, welche auch variabel gehalten werden kann. So lässt sich beispielsweise die Plasmaquelle mit der mindestens einen Reinigungskammer verbinden und/oder in die mindestens eine Reinigungskammer integrieren. Alternativ oder zusätzlich kann mindestens eine Desinfektionskammer vorgesehen sein, welche die Plasmaquelle aufweist und/oder welche mit der Plasmaquelle verbunden ist. Alternativ oder zusätzlich kann die Plasmaquelle auch als separates Gerät ausgestaltet sein, in welches beispielsweise eine Plasmaquelle in Form z.B. einer Plasmadusche integriert sein kann. Weiterhin lässt sich die Plasmaquelle auch derart ausgestalten, dass Bedienelemente der Reinigungsvorrichtung mittels des Reaktivgases keimfrei gehalten werden. Beispielsweise kann eine Plasmadusche an einem Türgriff und/oder an einem Bedienpanel einer Reinigungsvorrichtung vorgesehen sein, beispielsweise einer Bedienfolie, beispielsweise um dieses Bedienpanel keimfrei zu halten und/oder zu desinfizieren. Insgesamt ist die erfindungsgemäße Idee, eine Reinigungsvorrichtung mit mindestens einer Plasmaquelle zur Erzeugung mindestens eines Reaktivgases auszugestalten, somit sehr flexibel und universell im Bereich der Reinigungstechnik einsetzbar.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Gleiche Bezugsziffern in den einzelnen Figuren bezeichnen dabei gleiche und/oder funktionsgleiche bzw. hinsichtlich ihrer Funktionen einander entsprechende Elemente.

Es zeigen:
- Figur 1: ein Ausführungsbeispiel einer nicht beanspruchten Einkammer-Spülmaschine, insbesondere einer Einkammer-Geschirrspülmaschine;
- Figur 2: ein Ausführungsbeispiel einer nicht beanspruchten Durchlaufgeschirrspülmaschine;
- Figur 3: ein Ausführungsbeispiel eines erfindungsgemäßen Reinigungs- und Desinfektionsgeräts; und
- Figuren 4A bis 4C: verschiedene Positionen eines nicht beanspruchten Ausführungsbeispiels einer zur Reinigung von Lebensmitteln eingerichteten Reinigungsvorrichtung.

### Ausführungsbeispiele

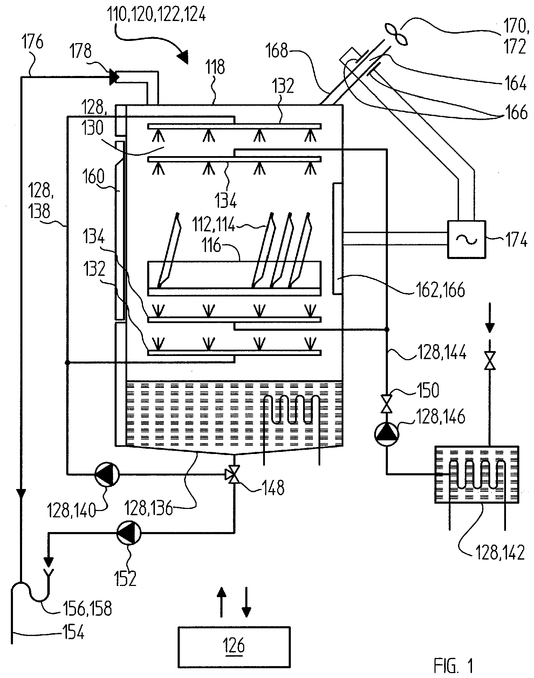

In Figur 1 ist ein erstes Ausführungsbeispiel einer nicht beanspruchten Reinigungsvorrichtung 110 zur Reinigung von Reinigungsgut 112 dargestellt. Das Reinigungsgut 112 ist exemplarisch in Form von Geschirr 114 dargestellt. Alternativ oder zusätzlich kommen jedoch auch andere Arten von Reinigungsgut 112 in Betracht, beispielsweise, wie oben ausgeführt, Gegenstände einer persönlichen Schutzausrüstung und/oder andere Arten von Reinigungsgut. Das Reinigungsgut kann beispielsweise in einer Aufnahme 116 im Inneren einer Reinigungskammer 118 aufgenommen sein, beispielsweise in mindestens einem Korb, beispielsweise in mindestens einem Geschirrkorb. Die Reinigungsvorrichtung 110 kann somit in dem in Figur 1 dargestellten Ausführungsbeispiel insbesondere als Einkammer-Spülmaschine 120 ausgestaltet sein, insbesondere als Einkammer-Geschirrspülmaschine 122. Die Reinigungsvorrichtung 110 kann derart ausgestaltet sein, dass ein Reinigungsprogramm mit optional mehreren Reinigungsprogrammschritten durchgeführt wird, sodass die Reinigungsvorrichtung 110 beispielsweise als Programmautomat 124 ausgestaltet sein kann. Die Reinigungsvorrichtung 110 kann zu diesem Zweck beispielsweise mindestens eine Ansteuerung 126 umfassen, beispielsweise mit mindestens einer Datenverarbeitungsvorrichtung, optional mit mindestens einer Benutzerschnittstelle (nicht dargestellt), welche beispielsweise programmtechnisch eingerichtet sein kann, um das Reinigungsprogramm mit den verschiedenen Reinigungsprogrammschritten anzusteuern. Dies ist in Figur 1 symbolisch dargestellt.

Die Reinigungsvorrichtung 110 weist, wie oben ausgeführt, eine Reinigungskammer 118 auf. In dieser Reinigungskammer ist als Bestandteil einer Fluidquelle 128 ein Düsensystem 130 vorgesehen. Dieses Düsensystem 130 kann beispielsweise, wie in Figur 1 dargestellt, einen oder mehrere Sprüharme umfassen. Beispielsweise kann das Düsensystem 130 oberhalb des Reinigungsguts 112 angeordnete Düsenarme und/oder unterhalb des Reinigungsguts 112 angeordnete Düsenarme umfassen. Dabei können auch unterschiedliche Düsensysteme 130 für die Beaufschlagung mit unterschiedlichen Arten von Reinigungsfluid vorgesehen sein. Exemplarisch ist in Figur 1 ein Fall dargestellt, bei welchem ein Spüldüsensystem 132 mit Sprüharmen jeweils oberhalb und unterhalb des Reinigungsguts 112 vorgesehen ist, und ein Nachspüldüsensystem 134, ebenfalls mit Sprüharmen oberhalb und unterhalb des Reinigungsguts 112. Auch andere Ausgestaltungen sind jedoch grundsätzlich möglich. Die Einkammer-Spülmaschine 120 gemäß Figur 1 kann beispielsweise, wie in Figur 1 dargestellt, als Mehrtank-System ausgestaltet sein und kann beispielsweise mindestens einen Waschtank 136 am Boden der Reinigungskammer 118 umfassen, aus welchem über ein Rohleitungssystem 138 als weiterer Bestandteil der Fluidquelle 128 und optional eine Pumpe 140 eine Beaufschlagung des Spüldüsensystems 132 mit Reinigungsfluid erfolgt. Zusätzlich kann, wie in Figur 1 dargestellt, mindestens ein Nachspültank 142 separat von dem Waschtank 136 ausgebildet sein, welcher hier exemplarisch als Boiler ausgestaltet ist.

Auch andere Ausgestaltungen sind jedoch grundsätzlich möglich, beispielsweise Ausgestaltungen, bei welchen eine Aufheizung einer Nachspülflüssigkeit über mindestens einen Durchlauferhitzer erfolgt. Der Nachspültank 142 kann, wie auch der Waschtank 136, ebenfalls Bestandteil der Fluidquelle 128 sein und kann über ein Rohrleitungssystem 144 und optional über eine Pumpe 146 verfügen, über welche das Nachspüldüsensystem 134 mit Reinigungsfluid in Form von Nachspülflüssigkeit, beispielsweise Frischwasser, optional unter Zusatz von Hilfsstoffen, beaufschlagt wird. Weiterhin kann die Fluidquelle 128 über ein oder mehrere Ventile verfügen, wobei in Figur 1 exemplarisch Ventile 148 und 150 dargestellt sind. Das Ventil 148 kann beispielsweise am Auslauf des Waschtanks 136 vorgesehen sein und kann beispielsweise als 3-Wege-Ventil ausgestaltet sein. Dieses kann beispielsweise, optional über eine weitere Pumpe 152, mit mindestens einem Abfluss 154 verbunden sein, vorzugsweise einem Abfluss 154 mit einem Geruchsverschluss 156, beispielsweise einem Siphonbogen 158. Die Ansteuerung 126 kann beispielsweise eingerichtet sein, um eine oder mehrere der Pumpen 140, 146, 152 und/oder eins oder mehrere der Ventile 148, 150 anzusteuern.

Das Reinigungsgut 112 kann beispielsweise durch mindestens eine Öffnung 160 in einen Innenraum der Reinigungskammer 118 einbringbar sein. Diese mindestens eine Öffnung 160 kann beispielsweise verschließbar ausgestaltet sein, insbesondere, wie in Figur 1 gezeigt, durch eine Klappe. Auch andere Verschlussmechanismen sind jedoch alternativ oder zusätzlich einsetzbar, beispielsweise Schwenktüren, Schieber, aufklappbare Teile einer Haube, verschiebbare Hauben oder andere Arten verschließbarer Öffnungen.

Die Reinigungsvorrichtung 110 gemäß dem Ausführungsbeispiel in Figur 1 umfasst weiterhin mindestens eine Plasmaquelle 162, 164. In dem dargestellten Ausführungsbeispiel sind zwei Möglichkeiten realisiert, welche einzeln oder in Kombination einsetzbar sind. So kann beispielsweise eine erste Plasmaquelle 162 vorgesehen sein, mit Elektroden 166, welche in Figur 1 lediglich angedeutet sind und welche innerhalb der Reinigungskammer 118 und/oder innerhalb einer Wand der Reinigungskammer 118 angeordnet sein können. Bezüglich der Ausgestaltung dieser Elektroden kann beispielsweise auf den obigen Stand der Technik verwiesen werden. Diese Plasmaquelle 162 kann beispielsweise eingerichtet sein, um in einem in dem Innenraum der Reinigungsvorrichtung 118 befindlichen Gas, beispielsweise Luft, insbesondere mit einem hohen Feuchtigkeitsanteil, ein Plasma zu zünden, wodurch wiederum ein Reaktivgas erzeugt wird, das vorzugsweise dieses Plasma umfasst.

Alternativ oder zusätzlich kann mindestens eine, außerhalb der Reinigungskammer 118 angeordnete, Plasmaquelle 164 vorgesehen sein, welche beispielsweise als Plasmaspender ausgestaltet sein kann. In dem in Figur 1 dargestellten Ausführungsbeispiel kann die Plasmaquelle 164 beispielsweise wiederum mit Elektroden 166 ausgestaltet sein. Beispielsweise kann diese Plasmaquelle 164, welche als Plasmaspender ausgestaltet sein kann, in einem Zuluftstutzen 168 angeordnet sein, welcher in die Reinigungskammer 118 mündet. Dieser Zuluftstutzen 168 kann beispielsweise mit einem Gebläse 170 ausgestattet und/oder verbunden sein, welches beispielsweise Bestandteil einer Druckvorrichtung 172, insbesondere zur Erzeugung eines Überdrucks, oder schlicht zur Zufuhr von Frischluft in die Reinigungskammer 118 ausgestaltet sein kann. Mittels der Plasmaquelle 164 kann beispielsweise in einem, in dem Zuluftstutzen 168 befindlichen, Gas ein Plasma gezündet werden, mittels dessen ein Reaktivgas erzeugt werden kann, das dann wiederum durch das Gebläse 170 ins Innere der Reinigungskammer 118 transferiert werden kann und dort beispielsweise gezielt auf das Reinigungsgut 112 aufgebracht werden kann. Die Plasmaquellen 162, 164 können beispielsweise jeweils einzeln oder gemeinsam mit mindestens einer elektrischen Energiequelle 174 verbunden sein, welche in Figur 1 lediglich angedeutet ist und welche beispielsweise eine Wechselspannungsquelle und/oder eine Wechselstromquelle umfassen kann. Die Plasmaquellen 162, 164 und/oder die elektrischen Energiequellen 174 können ebenfalls durch die Ansteuerung 126 kontrolliert und/oder angesteuert werden, sodass beispielsweise die Erzeugung des Plasmas ebenfalls durch die Ansteuerung 126 kontrolliert werden kann. Beispielsweise kann die Ansteuerung eingerichtet sein, um mindestens ein Reinigungsprogramm in der Reinigungsvorrichtung zu steuern, wobei in mindestens einem Reinigungsprogrammschritt des Reinigungsprogramms das Reinigungsgut 112 mit dem Reinigungsfluid beaufschlagt wird und wobei in mindestens einem Desinfektionsschritt des Reinigungsprogramms das Reinigungsgut 112 mit dem Reaktivgas beaufschlagt wird, vorzugsweise nach Durchführung des Reinigungsprogrammschritts, beispielsweise während eines Trocknungsschritts. Alternativ oder zusätzlich kann der Desinfektionsschritt auch beispielsweise, wie oben ausgeführt, vor einem Nachspülschritt oder Klarspülschritt durchgeführt werden, sodass beispielsweise in dem Nachspül- oder Klarspülschritt an dem Reinigungsgut 112 anhaftende Verunreinigungen und/oder Reste des Reaktivgases durch das Nachspülfluid entfernt werden können. Verschiedene andere Ausgestaltungen sind möglich. Zur Initiierung des Desinfektionsschritts kann die Ansteuerung 126 beispielsweise die elektrischen Energiequellen 174 entsprechend ansteuern und/oder das Gebläse 170 ansteuern.

Weiterhin kann die Reinigungsvorrichtung 110, wie ebenfalls in Figur 1 exemplarisch gezeigt, eingerichtet sein, um das Reaktivgas aus der Reinigungskammer 118 in den Abfluss 154 abzuführen. Dies kann beispielsweise durch eine Zwangsverdrängung aus der Reinigungskammer 118 erfolgen. Zu diesem Zweck kann beispielsweise ein Abluftkanal 176 vorgesehen sein, welcher die Reinigungskammer 118 mit dem Abfluss 154 verbindet, vorzugsweise hinter dem Geruchsverschluss 156, sodass der Geruchsverschluss 156 zwischen einer Einmündung des Abluftkanals 176 und einer Verbindung zur Reinigungskammer 118 angeordnet ist. Der Abluftkanal 176 kann beispielsweise ein oder mehrere Ventile umfassen, insbesondere, wie in Figur 1 angedeutet, mindestens ein Rückschlagventil 178, beispielsweise um zu verhindern, dass Gase und/oder Flüssigkeiten aus dem Abfluss 154 zurück in die Reinigungskammer 118 gelangen können. Die Reinigungsvorrichtung 110 kann beispielsweise derart eingerichtet sein, dass Reaktivgas, beispielsweise durch Einleiten von unter Druck stehender Zuluft und/oder Dampf, beispielsweise mittels des Gebläses 170, aus der Reinigungskammer 118 über den Abluftkanal 176 in den Abfluss 154 verdrängt werden. Diese Verdrängung kann beispielsweise wiederum durch die Ansteuerung 126 gesteuert werden, beispielsweise nach Beendigung eines Desinfektionsschritts eines Reinigungsprogramms.

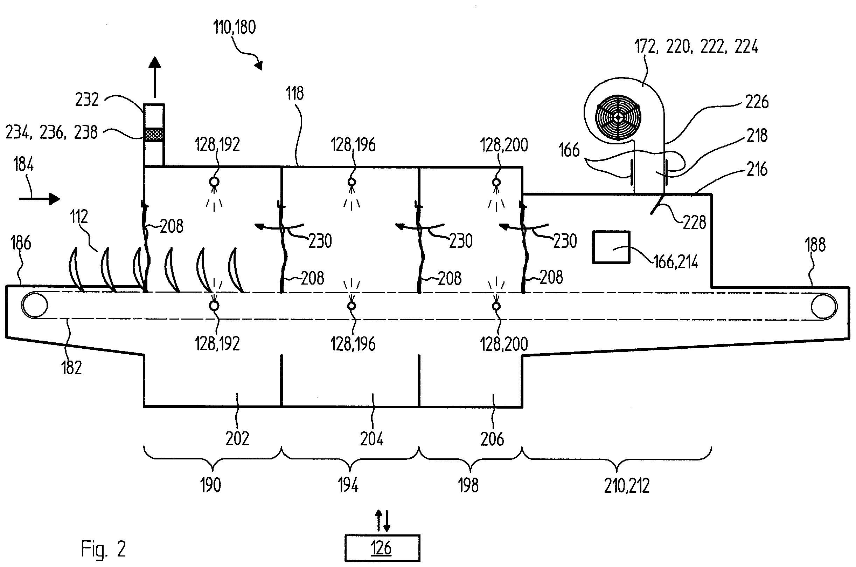

In Figur 2 ist ein weiteres Ausführungsbeispiel einer nicht beanspruchten Reinigungsvorrichtung 110 dargestellt. In diesem Ausführungsbeispiel ist die Reinigungsvorrichtung 110 als Durchlaufgeschirrspülmaschine 180 ausgestaltet. Die Durchlaufgeschirrspülmaschine umfasst wiederum eine Reinigungskammer 118, welche in diesem Ausführungsbeispiel exemplarisch als Tunnel ausgestaltet ist. Beispielsweise kann ein Transportsystem 182 vorgesehen sein, insbesondere ein Transportband, mittels dessen Reinigungsgut 112 in einer Transportrichtung 184 von einem Einlauf 186 zu einem Auslauf 188 befördert wird. Für mögliche Einzelheiten der Ausgestaltung der Durchlaufgeschirrspülmaschine 180 kann auf den oben beschrieben Stand der Technik verwiesen werden, beispielsweise auf die DE 10 2006 039 434 A1. Auch andere Ausgestaltungen sind jedoch grundsätzlich möglich. Die Durchlaufgeschirrspülmaschine 180 kann beispielsweise als Bandtransport- oder Korbtransport-Geschirrspülmaschine ausgestaltet sein.

Auch in dem in Figur 2 gezeigten Ausführungsbeispiel umfasst die Reinigungsvorrichtung 110 wiederum eine Fluidquelle 128, welche in diesem Ausführungsbeispiel eine Mehrzahl von Düsensystemen umfasst. So ist die Reinigungskammer 118 vorzugsweise in eine Mehrzahl von Zonen unterteilt, nämlich eine Vorabräumzone 190 mit einem Vorabräum-Düsensystem 192, mindestens eine Hauptreinigungszone 194 mit mindestens einem Hauptreinigungs-Düsensystem 196 und mindestens eine Nachspülzone 198, welche auch als Klarspülzone ausgestaltet und/oder bezeichnet werden kann, mit mindestens einem Nachspül-Düsensystem 200, beispielsweise mindestens einem Pumpenklarspül-Düsensystem und/oder mindestens einem Frischwasserklarspül-Düsensystem. Die Düsensysteme 192, 196 und 200 können beispielsweise über in Figur 2 nicht dargestellte Pumpen aus entsprechenden Tanks 202 bis 206 mit Reinigungsfluid beaufschlagt werden. Die Reinigungszonen 190, 194, 198 können untereinander und/oder nach außen jeweils durch Vorhänge 208 abgetrennt sein. An die Reinigungszonen 190, 194, 198 kann sich beispielsweise mindestens eine Trocknungszone 210 anschließen, welche in diesem Ausführungsbeispiel optional gleichzeitig auch als Desinfektionszone 212 ausgestaltet sein kann. Alternativ oder zusätzlich kann eine Desinfektion jedoch auch in einer separaten Desinfektionszone erfolgen und/oder eine Desinfektionszone 212 kann mit einer oder mehreren der Reinigungszonen 190, 194 oder 198 zusammengefasst sein.

Die Reinigungsvorrichtung 110 weist wiederum mindestens eine Plasmaquelle 214 auf, beispielsweise wiederum mit mindestens zwei Elektroden 166, wie in Figur 2 angedeutet. Die Elektroden 166 können beispielsweise wiederum, ähnlich zur Ausgestaltung gemäß Figur 1, in einer Wand der Reinigungskammer 118 und/oder einer die Desinfektionszone 212 umfassenden Desinfektionskammer 216, welche separat von der Reinigungskammer 118 ausgestaltet sein kann und/oder auch ganz oder teilweise mit derselben identisch sein kann, angeordnet sein. Alternativ oder zusätzlich kann wiederum, wie in Figur 2 ebenfalls angedeutet, mindestens eine Plasmaquelle 218, beispielsweise wiederum mit Elektroden 166, außerhalb der Reinigungskammer 118 und/oder außerhalb der Desinfektionskammer 216 angeordnet sein, beispielsweise als separater Plasmaspender. So kann beispielsweise wiederum mindestens ein Gebläse 220 vorgesehen sein, beispielsweise als Bestandteil einer Druckvorrichtung 172 und/oder als Bestandteil einer Trocknungsvorrichtung 222 und/oder als separate Vorrichtung. In Figur 2 ist exemplarisch, ohne Beschränkung weiterer möglicher Ausgestaltungen, das Gebläse 220 als Trocknungsgebläse 224 ausgestaltet, welches über einen Zuluftstutzen 226 mit der Trocknungszone 210 verbunden sein kann, um das Reinigungsgut 112 in dieser Trocknungszone 210 mit Luft zu beaufschlagen, vorzugsweise mit erwärmter Luft. Die Plasmaquellen 214, 218 können wiederum eingerichtet sein, um ein Plasma in einem Gas zu erzeugen, wodurch wiederum ein Reaktivgas, vorzugsweise enthaltend das Plasma oder einen Teil desselben, erzeugt wird.

Bei der Plasmaquelle 214 kann beispielsweise das Plasma unmittelbar in der Desinfektionskammer 216 erzeugt werden und/oder, bei Anordnung in einer oder mehreren der Reinigungskammern 190, 194, 198, in dieser Reinigungskammer. Bei der Plasmaquelle 218 kann beispielsweise das Plasma in dem Zuluftstutzen 226 erzeugt werden und dann beispielsweise mittels eines durch das Gebläse 220 erzeugten Zuluftstroms auf das Reinigungsgut 112 aufgebracht werden. Der Zuluftstrom und, mit diesem, das Reaktivgas, welches durch die Plasmaquelle 218 erzeugt wird, können beispielsweise mittels eines oder mehrerer Strömungsleitelemente 228 in ihrer Richtung beeinflusst werden, beispielsweise um dem Zuluftstrom eine Richtung entgegen der Transportrichtung 184 zu verleihen. Auf diese Weise kann beispielsweise innerhalb der Reinigungsvorrichtung 110 ein Luftstrom 230 erzeugt werden, der der Transportrichtung 184 entgegengesetzt gerichtet ist. Mit diesem Luftstrom kann beispielsweise auch das Reaktivgas entgegengesetzt zur Transportrichtung 184 transportiert werden.

Weiterhin kann die Reinigungsvorrichtung 110, wie in Figur 2 ebenfalls angedeutet, mindestens eine Absaugung 232 umfassen, beispielsweise im Bereich des Einlaufs 186. Diese Absaugung 232 kann beispielsweise mit einem Abluftstutzen (in Figur 2 nicht dargestellt) verbunden sein, beispielsweise in einem Dachbereich eines Gebäudes, in welchem die Reinigungsvorrichtung 110 gemäß Figur 2 angeordnet ist. Innerhalb der Absaugung 232, welche beispielsweise wiederum ein Gebläse umfassen kann, kann insbesondere mindestens eine Aufbereitungsvorrichtung 234 vorgesehen sein, beispielsweise mindestens ein Filter 236 und/oder mindestens ein Katalysator 238. Auf diese Weise können beispielsweise das Reaktivgas und/oder Bestandteile desselben aus einem Abluftstrom entfernt und/oder in unkritischere Komponenten umgewandelt werden. Eine derartige Aufbereitungsvorrichtung 234 kann optional auch in anderen Ausgestaltungen der Reinigungsvorrichtung 110 vorhanden sein, beispielsweise in der Reinigungsvorrichtung 110 gemäß Figur 1, beispielsweise in dem Abluftkanal 176.

Die Reinigungsvorrichtung 110 gemäß der Ausgestaltung in Figur 2 kann optional wiederum mindestens eine Ansteuerung 126 umfassen. Diese Ansteuerung 126 kann beispielsweise das Transportsystem 182 ansteuern und/oder die Fluidquelle 128 und/oder die Plasmaquellen 214, 218. Die Plasmaquellen 214, 218 können wiederum, was in Figur 2 nicht dargestellt ist, mit einer oder mehreren elektrischen Energiequellen 174 verbunden sein, beispielsweise analog zur Ausgestaltung gemäß Figur 1. Die Ausgestaltung der optionalen Elektroden 166 kann beispielsweise wiederum gemäß dem oben beschriebenen Stand der Technik erfolgen.

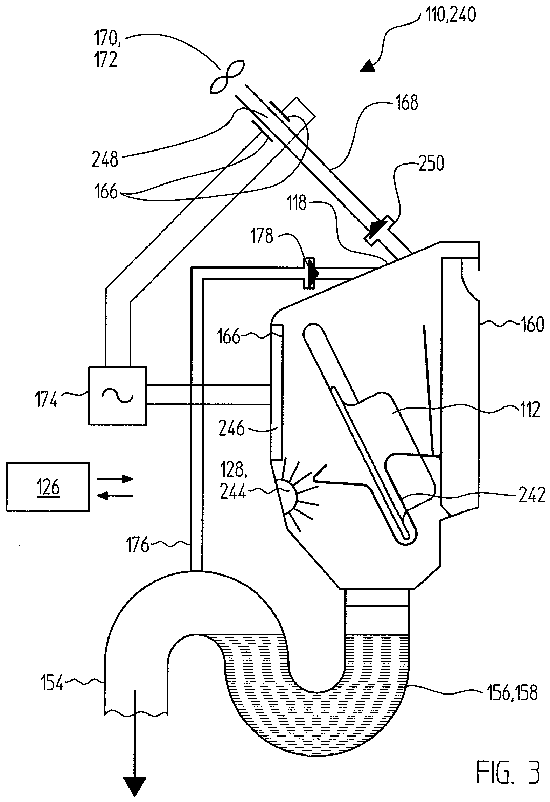

In Figur 3 ist ein weiteres Ausführungsbeispiel einer erfindungsgemäßen Reinigungsvorrichtung 110, in einer zu den Figuren 1 und 2 analogen Schnittdarstellung, dargestellt. Die Reinigungsvorrichtung 110 ist in diesem Ausführungsbeispiel als Reinigungs- und Desinfektionsgerät 240 ausgestaltet und ist beispielsweise zur Reinigung von Reinigungsgut 112 in Form von Steckbecken, Urinflaschen oder ähnlichem ausgestaltet. Die Reinigungsvorrichtung 110 weist wiederum eine Reinigungskammer 118 auf, in deren Innenraum das Reinigungsgut 112 aufnehmbar ist. Beispielsweise kann die Reinigungskammer 118 wiederum, ähnlich zur Ausgestaltung gemäß Figur 1, eine verschließbare Öffnung 160 aufweisen, beispielsweise wiederum mit einer Klappe. Eine Halterung 242 als Aufnahme für das Reinigungsgut 112 kann beispielsweise mit der Klappe verbunden sein, sodass bei einem Verschließen der Reinigungskammer 118 eine Entleerung des Reinigungsguts 112 erfolgt, sodass feste und/oder flüssige Abfälle aus dem Inneren des Reinigungsguts 112 entleert werden können. Zu diesem Zweck kann die Reinigungsvorrichtung 110 mindestens einen Abfluss 154 aufweisen, welcher beispielsweise von seinem Durchmesser her derart groß ausgestaltet ist, das die Ausscheidungen und insbesondere größere Mengen an Flüssigkeit problemlos aufgenommen werden können. Der Abfluss 154 kann beispielsweise wiederum mit einem Geruchsverschluss 156 ausgestaltet sein, insbesondere einem Siphonbogen 158.

Die Reinigungsvorrichtung 110 gemäß dem Ausführungsbeispiel in Figur 3 umfasst wiederum mindestens eine Fluidquelle 128, insbesondere in Form mindestens eines Düsensystems 244. Über dieses Düsensystem 244 kann das Reinigungsgut 112 beispielsweise mit Reinigungsfluid in Form von Flüssigkeit und/oder Dampf beaufschlagt werden. Weitere Bestandteile der Fluidquelle 128, beispielsweise ein oder mehrere Flüssigkeitstanks und/oder eine oder mehrere Pumpen und/oder ein oder mehrere Dampferzeuger, sind in Figur 3 zur Vereinfachung nicht dargestellt. Für weitere mögliche Ausgestaltungen der Reinigungsvorrichtung 110 kann beispielsweise auf den oben beschriebenen Stand der Technik verwiesen werden, insbesondere auf DE 103 48 344 B4 und/oder EP 1 824 373 B1.

Die Reinigungsvorrichtung 110 kann wiederum über eine oder mehrere Plasmaquellen 246, 248 verfügen, beispielsweise wiederum mit jeweils Elektroden 166, beispielsweise gemäß der oben beschriebenen Ausgestaltung. Die Plasmaquellen 246, 248 können weiterhin wiederum mit einer oder mehreren elektrischen Energiequellen 174 verbunden sein. Wiederum sind die bereits anhand des Beispiels in Figur 1 erläuterten Möglichkeiten dargestellt, welche einzeln oder auch in Kombination realisierbar sind. So ist die mindestens eine Plasmaquelle 246 gemäß Figur 3 optional vollständig oder teilweise im Inneren der Reinigungskammer 118 angeordnet, sodass ein Plasma unmittelbar im Inneren der Reinigungskammer 1118 gezündet werden kann. Alternativ oder zusätzlich ist die mindestens eine Plasmaquelle 248 vorgesehen, welche ein Plasma in einem Zuluftstutzen 168 erzeugen kann, welcher optional mit mindestens einem Gebläse 170 als Bestandteil einer Druckvorrichtung 172 ausgestattet und/oder verbunden sein kann. Die Plasmaquelle 248 wirkt somit als Plasmaspender und erzeugt das Plasma beispielsweise in einem Gas innerhalb des Zuluftstutzens 168, welches dann beispielsweise mittels der Druckvorrichtung 172, insbesondere des Gebläses 170, in die Reinigungskammer 118 eingebracht werden kann, insbesondere unter Überdruck. Der Zuluftstutzen 168 kann beispielsweise auch wiederum mit mindestens einem Ventil 250 versehen sein. Diese Ausgestaltung kann auch bei dem Ausführungsbeispiel gemäß Figur 1 oder bei anderen Ausführungsbeispielen der Reinigungsvorrichtung 110 realisierbar sein.

Die Reinigungsvorrichtung 110 kann wiederum mindestens eine Ansteuerung 126 umfassen, welche beispielsweise eingerichtet sein kann, um die Fluidquelle 128 und/oder die Plasmaquellen 246, 248 anzusteuern. Weiterhin kann auch beispielsweise das Gebläse 170 durch die Ansteuerung 126 angesteuert werden. Die Ansteuerung 126 kann wiederum, analog beispielsweise zur Ausgestaltung gemäß Figur 1, eingerichtet sein, um mindestens ein Reinigungsprogramm durchzuführen, beispielsweise mit mindestens einem Reinigungsprogrammschritt, in welchem das Reinigungsgut 112 mit Reinigungsfluid beaufschlagt wird, und mindestens einem Desinfektionsschritt, in welchem das Reinigungsgut 112 mit dem Plasma bzw. dem Reaktivgas, welches durch eine oder mehrere der Plasmaquellen 246, 248 erzeugt wird, beaufschlagt wird.

Analog zur Ausgestaltung gemäß Figur 1 kann die Reinigungsvorrichtung 110 in dem Ausführungsbeispiel gemäß Figur 3 auch wiederum mindestens einen Abluftkanal 176 umfassen, optional wiederum mit mindestens einem Ventil 178, beispielsweise mindestens einem Rückschlagventil. Der Abluftkanal 176 kann beispielsweise Reaktivgas und/oder andere Gasbestandteile und/oder Dampf aus der Reinigungskammer 118 in den Abfluss 154 überführen. Vorzugsweise liegt wiederum eine Mündung des Abluftkanals 176 jenseits des Geruchsverschlusses 156, beispielsweise jenseits des Siphonbogens 158, sodass der Geruchsverschluss 156 zwischen dieser Mündung und der Reinigungskammer 118 angeordnet ist. Beispielsweise durch die Druckvorrichtung 172 kann somit das Reaktivgas und/oder Bestandteile desselben über den Abluftkanal 176 in den Abfluss 154 verdrängt werden. Alternativ oder zusätzlich kann, analog zum Ausführungsbeispiel gemäß Figur 1, auch wiederum eine Auswaschung des Reaktivgases und/oder eine andere Art und Weise der Abfuhr erfolgen. Beispielsweise kann in dem Ausführungsbeispiel gemäß Figur 3, wie auch in dem Ausführungsbeispiel gemäß Figur 1, mindestens eine Entlüftung und/oder Absaugung vorgesehen sein, optional wiederum mit mindestens einer Aufbereitungsvorrichtung 234, analog zu dem Ausführungsbeispiel gemäß Figur 2.

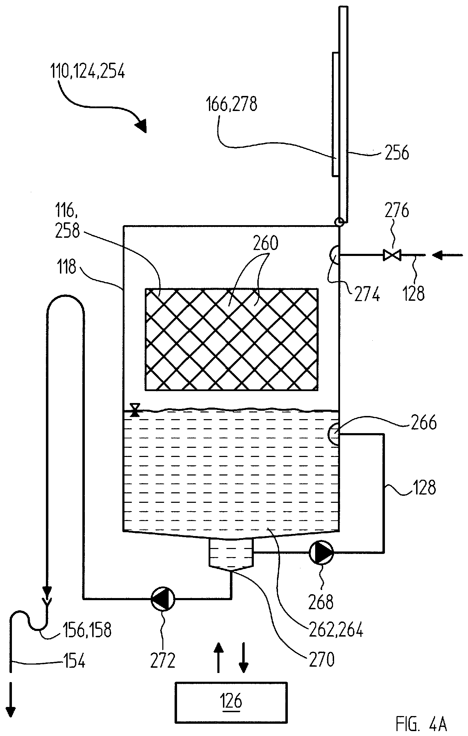

In den Figuren 4A bis 4C ist ein Ausführungsbeispiel einer Reinigungsvorrichtung 110 dargestellt, welche zur Reinigung von Reinigungsgut 112 in Form von Lebensmitteln 252 eingerichtet ist. Bei den Lebensmitteln kann es sich beispielsweise, wie oben ausgeführt, um Gemüse, Salat, Obst, Beeren, Knollenfrüchte oder ähnliche Lebensmittel handeln, insbesondere um pflanzliche Lebensmittel. Die Reinigungsvorrichtung 110 kann somit insbesondere als Gemüsewaschmaschine 254 ausgestaltet sein.

Die Reinigungsvorrichtung 110 weist in dem dargestellten Ausführungsbeispiel wiederum eine Reinigungskammer 118 auf, welche in diesem Ausführungsbeispiel exemplarisch von oben beladen werden kann. Die Reinigungsvorrichtung 110 ist somit beispielsweise als Toplader ausgestaltet und weist zu diesem Zweck beispielsweise einen Deckel 256 auf, welcher aufklappbar ist, um die Reinigungskammer 118 mit dem Reinigungsgut 112 zu beladen.

Die Reinigungsvorrichtung 110 weist wiederum eine Aufnahme 116 für das Reinigungsgut 112 auf. In diesem Ausführungsbeispiel kann es sich bei der Aufnahme 116 insbesondere um einen Korb 258 handeln, beispielsweise einen aus mindestens einem metallischen Material und/oder aus mindestens einem Kunststoffmaterial hergestellten Korb 258. Die Aufnahme 116 kann ganz oder teilweise aus einem Kunststoffmaterial und/oder aus einem metallischen Material hergestellt sein. Die Aufnahme 116 ist eingerichtet, um die Lebensmittel 252 zurückzuhalten während eines Reinigungsvorgangs, jedoch gleichzeitig ein Abfließen und vorzugsweise auch ein Eindringen von Reinigungsfluid zu ermöglichen, beispielsweise durch eine Mehrzahl von Öffnungen 260, welche entsprechend dimensioniert sind. Der Korb 258 kann beispielsweise in den Figuren 4A bis 4C nach oben geöffnet und nach unten geschlossen sein. Der Korb kann beispielsweise rund ausgestaltet sein, beispielsweise durch eine vertikale Achse in der Zeichenebene in den Figuren 4A bis 4C.

Die Reinigungsvorrichtung 110 in dem in den Figuren 4A bis 4C dargestellten Ausführungsbeispiel kann beispielsweise wiederum als Einkammer-Maschine ausgestaltet sein. Grundsätzlich sind jedoch auch Ausgestaltungen mit mehr als einer Reinigungskammer 118 möglich.

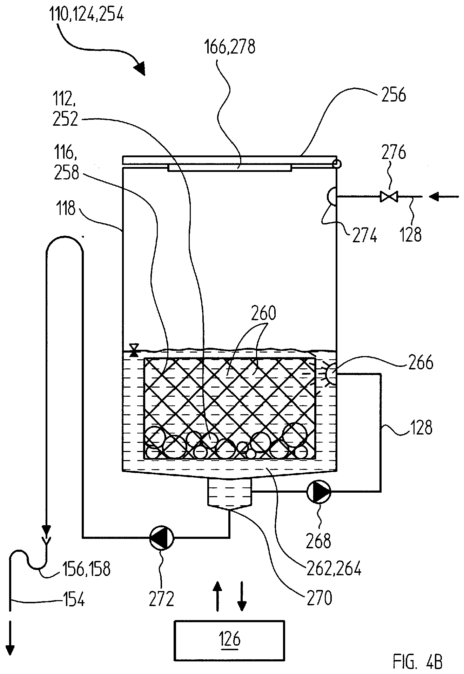

Die Reinigungsvorrichtung 110 kann beispielsweise, insbesondere innerhalb der Reinigungskammer 118, mindestens ein Reinigungsbad 262 aufweisen, in welches die Lebensmittel 252 mittels der Aufnahme 116 eingetaucht werden können. In dem dargestellten Ausführungsbeispiel ist das Reinigungsbad 262 am Boden der Reinigungskammer 118 in Form eines Waschtanks 264 ausgebildet. Die Reinigungsvorrichtung 110 kann insbesondere derart ausgestaltet sein, dass das Lebensmittel 252 in mindestens einer ersten Position innerhalb des Reinigungsbads 262 angeordnet ist und in mindestens einer weiteren Position außerhalb des Reinigungsbads 262. Dies ist in den Figuren 4A bis 4C dargestellt. So zeigt Figur 4A eine Position, welche hier im Folgenden auch als "zweite Position" bezeichnet wird (ohne durch diese Namensgebung eine Reihenfolge vorzugeben), in welcher der Deckel 256 geöffnet ist und die Reinigungsvorrichtung 110 mit dem Lebensmittel 252 beladen werden kann. Es handelt sich bei der in Figur 4A dargestellten Position somit um eine Position zum Beladen und/oder Entladen der Reinigungsvorrichtung 110 mit dem Reinigungsgut 112. In Figur 4B ist hingegen eine Position gezeigt, welche hier und im Folgenden auch als "erste Position" bezeichnet wird, in welcher die Aufnahme 116 mit dem Lebensmittel 252 beladen ist und in welcher das Lebensmittel 252 in das Reinigungsbad 262 eintaucht. Beispielsweise kann dieses Eintauchen vollständig erfolgen. In dieser eingetauchten Position gemäß Figur 4B kann die Aufnahme 116 auch bewegbar gelagert sein, beispielsweise drehbar gelagert. So kann beispielsweise die Aufnahme 116 um eine in Figur 4B nicht dargestellte, vertikal in der Zeichenebene verlaufende Achse drehbar gelagert sein, beispielsweise um eine Achse senkrecht zur Flüssigkeitsoberfläche des Reinigungsfluids in dem Reinigungsbad 262. Diese Rotation kann beispielsweise durch die Reinigungsvorrichtung 110 mittels mindestens eines Motors angetrieben werden und/oder fluidisch. So kann beispielsweise in dem Reinigungsbad 262 mindestens ein Waschdüsensystem 266 vorgesehen sein, welches beispielsweise aus dem Waschtank 264 über mindestens eine Waschpumpe 268 mit Reinigungsfluid beaufschlagbar ist. Dieses Waschdüsensystem 266 kann beispielsweise mit einer tangentialen Komponente auf und/oder in die Aufnahme 116 einstrahlen, so dass das Lebensmittel 252 in der Aufnahme 116 mit Reinigungsfluid und/oder einer verstärkten Strömung und/oder Strahlung des Reinigungsfluids beaufschlagt wird. Gleichzeitig kann optional diese Strahlung und/oder Strömung des Reinigungsfluids eine Rotation der Aufnahme 116 antreiben. Beispielsweise kann die Aufnahme 116 zu diesem Zweck Flügel aufweisen, welche von dem Reinigungsfluid angetrieben werden. Auf diese Weise kann beispielsweise das Lebensmittel 252, eingetaucht in das Bad des Reinigungsfluids, beispielsweise in ein Wasserbad, langsam gedreht werden und dabei von Reinigungsfluid umströmt und/oder durchströmt werden. Optional kann eine Hubeinrichtung vorgesehen sein, beispielsweise als Bestandteil des oben genannten Aktormechanismus, welche die Aufnahme 116 regelmäßig oder unregelmäßig, beispielsweise periodisch, aus dem Reinigungsbad 262 heraushebt, wobei anhaftende Verunreinigungen abgelöst und ausgetragen werden können. Diese Umströmung des Lebensmittels 252, kombiniert von der optionalen Auf- und Abbewegung, kann eine intensive Reinigung bei gleichzeitiger Schonung der empfindlichen Lebensmittel 252 bewirken. Der Waschtank 264 kann beispielsweise über einen Ablauf 270 und eine optionale Entleerungspumpe 272 entleerbar sein, beispielsweise in einen Abfluss 154, welcher optional wiederum mit einem Geruchsverschluss 156 in Form eines Siphonbogens 158 ausgestaltet sein kann.

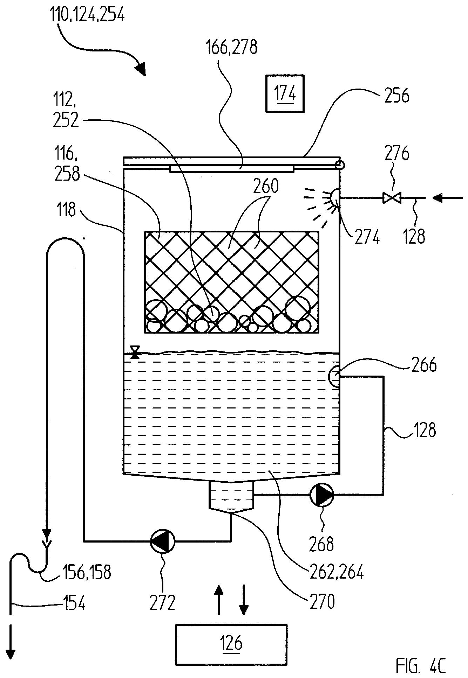

Während die in Figur 4B gezeigte Position, in welcher sich die Aufnahme 116 in der ersten Position befindet, als Reinigungsposition bezeichnet werden kann, ist in Figur 4C schließlich eine Position gezeigt, welche auch als Klarspülposition oder allgemein als Nachbehandlungsposition bezeichnet werden könnte. In dieser Position befindet sich die Aufnahme 116 in einer zweiten Position, in welcher das Lebensmittel 252 nicht mehr in das Reinigungsbad 262 eintaucht. Beispielsweise kann zu diesem Zweck die Aufnahme 116 aus dem Reinigungsbad 262 herausgehoben werden, beispielsweise wiederum mittels eines nicht dargestellten Aktormechanismus. Die Stellung der Aufnahme 116 in Figur 4C kann somit der Stellung der Aufnahme 116 in Figur 4A entsprechen. Der Deckel 256 kann dabei, wie in Figur 4C gezeigt, geschlossen sein.

In dieser Position kann das Lebensmittel 252 nacheinander oder gleichzeitig einer oder mehreren Behandlungen unterzogen werden, beispielsweise einer oder mehreren Nachbehandlungen. Eine optionale Möglichkeit, die in Figur 4C dargestellt ist, ist eine Behandlung über mindestens ein Spüldüsensystem 274, welches beispielsweise, analog zum Waschdüsensystem 266, in einer Kammerwand der Reinigungskammer 118 angeordnet sein kann. Wiederum kann mittels dieses Spüldüsensystems 274 beispielsweise eine Beaufschlagung des Lebensmittels 252 mit einem Reinigungsfluid erfolgen, welche vorzugsweise eine tangentiale Komponente hat. So kann auch in der in Figur 4C dargestellten zweiten Position der Aufnahme 116 beispielsweise eine Rotation der Aufnahme 116 erfolgen, angetrieben beispielsweise durch einen Motor und/oder durch das Reinigungsfluid mittels des Spüldüsensystems 274. Das Reinigungsfluid des Spüldüsensystems 274 kann beispielsweise Frischwasser sein, dessen Zufluss durch ein Ventil 276, beispielsweise ein Frischwasserventil, gesteuert werden kann. Beispielsweise kann das Reinigungsfluid ein Spülfluid, insbesondere Frischwasser, umfassen, welches wiederum vorzugsweise durch die Öffnungen 260 in die Aufnahme 116 eindringen kann, um das Lebensmittel 252 nachzuspülen. So kann beispielsweise eine Frischwassernachspülung vorgesehen sein, vorzugsweise mit klarem Leitungswasser.

An diese Nachspülung in der in Figur 4C dargestellten zweiten Position kann sich optional ein Schleudervorgang anschließen, bei welchem beispielsweise die Aufnahme 116 rotiert und anhaftendes Reinigungsfluid durch Fliehkräfte entfernt wird. Dieser Schleudervorgang kann beispielsweise wiederum motorisch angetrieben werden oder auch durch einen Fluidantrieb, beispielsweise mittels des Spüldüsensystems 274.

Vor, während oder nach dem einen oder mehreren der zuvor genannten Schritte, welche, mit Ausnahme des Schleuderschritts, als Reinigungsprogrammschritte bezeichnet werden können, schließt sich weiterhin mindestens ein Desinfektionsschritt an. Zu diesem Zweck weist die Reinigungsvorrichtung 110 wiederum mindestens eine Plasmaquelle 278 auf. In den in den Figuren 4A bis 4C gezeigten Ausführungsbeispielen ist diese Plasmaquelle 278 exemplarisch an einer der Reinigungskammer 118 zuweisenden Innenseite des Deckels 256 aufgenommen. Alternativ oder zusätzlich sind jedoch auch andere Ausgestaltungen möglich, beispielsweise durch Anbringung an einer oder mehreren Innenwänden der Reinigungskammer 118 und/oder in Form eines oder mehrerer Plasmaspender, welche außerhalb der Reinigungskammer angeordnet sein können. Bezüglich dieser Möglichkeiten kann auf die obige Beschreibung der übrigen Ausführungsbeispiele verwiesen werden. Die dort dargestellten Möglichkeiten der Ausgestaltung der Plasmaquelle sind grundsätzlich, einzeln oder in Kombination, auch im Rahmen der in den Figuren 4A bis 4C dargestellten Gemüsewaschmaschine 254 einsetzbar. Beispielsweise kann, wie in Figur 4C gezeigt, die Plasmaquelle 278 wiederum eine oder mehrere Elektroden 166 umfassen, beispielsweise an der Innenseite des Deckels 256. Alternativ oder zusätzlich können auch eine oder mehrere Elektroden 166 beispielsweise in einem in den Figuren 4A bis 4C nicht dargestellten Zuluftstutzen angeordnet sein. Die Plasmaquelle 278 kann wiederum optional mindestens eine elektrische Energiequelle 174 umfassen, wie in Figur 4C optional dargestellt. Der Desinfektionsschritt mittels der Plasmaquelle 278 kann in der in Figur 4C gezeigten zweiten Position der Aufnahme 116 erfolgen und/oder in einer separaten Desinfektionsposition. Verschiedene Ausgestaltungen sind denkbar.

Die Reinigungsvorrichtung 110 kann wiederum eine oder mehrere Ansteuerungen 126 umfassen, wie in den Figuren 4A bis 4C angedeutet, und kann insbesondere als Programmautomat 124 ausgestaltet sein. Auch andere Ausgestaltungen sind jedoch grundsätzlich möglich. Mittels der Ansteuerung 126 kann beispielsweise ein Reinigungsprogramm in der Reinigungsvorrichtung 110 gesteuert werden, welches beispielsweise zunächst ein Beladen der Aufnahme 116 mit dem Lebensmittel 252 ermöglicht, beispielsweise in der in Figur 4A gezeigten Stellung. Anschließend kann mindestens ein Reinigungsprogrammschritt durchgeführt werden. Dieser mindestens eine Reinigungsprogrammschritt kann beispielsweise mindestens einen Waschschritt umfassen, beispielsweise gemäß der obigen Beschreibung der Figur 4B. An diesen mindestens einen Waschschritt kann sich optional mindestens ein Reinigungsprogrammschritt in Form mindestens eines Spülschritts anschließen, beispielsweise in Form mindestens einer Frischwassernachspülung, beispielsweise gemäß der obigen Beschreibung der Figur 4C. An diesen mindestens einen optionalen Nachspülschritt kann sich optional mindestens ein Trocknungsschritt anschließen, beispielsweise der oben genannte mindestens eine optionale Schleuderschritt. Dieser kann ebenfalls beispielsweise gemäß der obigen Beschreibung der Figur 4C durchgeführt werden. Weiterhin kann die Ansteuerung 126 eingerichtet sein, um vor, während oder nach dem mindestens einen Reinigungsprogrammschritt und/oder zwischen mehreren Reinigungsprogrammschritten mindestens einen Desinfektionsschritt durchzuführen. Insbesondere kann dies in der in Figur 4C dargestellten Stellung erfolgen. In diesem mindestens einen Desinfektionsschritt wird das Lebensmittel 252 mit dem durch die mindestens eine Plasmaquelle 278 erzeugten Reaktivgas beaufschlagt, beispielsweise indem dieses Reaktivgas das Lebensmittel 252 umspült oder durchspült. Zu diesem Zweck kann das Reaktivgas beispielsweise von oben und/oder durch die Öffnungen 260 in den Korb 258 eindringen und das Lebensmittel 252 umspülen und/oder durchspülen. Auf diese Weise kann eine schonende Desinfektion des Lebensmittels 252 erzielt werden, vorzugsweise ohne Verwendung chemischer Desinfektionsmittel und/oder Wärme.

## Patentansprüche

1. Reinigungsvorrichtung (110) zur Reinigung von Reinigungsgut (112), wobei die Reinigungsvorrichtung (110) eine Reinigungskammer (118) zur Aufnahme des Reinigungsguts (112) aufweist, wobei die Reinigungsvorrichtung (110) in der Reinigungskammer (118) mindestens eine Fluidquelle (128) zur Beaufschlagung des Reinigungsguts (112) mit mindestens einem Reinigungsfluid aufweist, wobei die Reinigungsvorrichtung (110) weiterhin mindestens eine Plasmaquelle (162, 164, 214, 218, 246, 248, 278) aufweist, wobei die Plasmaquelle (162, 164, 214, 218, 246, 248, 278) eingerichtet ist, um mindestens ein Plasma in mindestens einem Gas zu zünden und mindestens ein Reaktivgas zu erzeugen, wobei die Reinigungsvorrichtung (110) eingerichtet ist, um das Reaktivgas mit zumindest einem Teil der Reinigungsguts (112) in Kontakt zu bringen, wobei die Reinigungsvorrichtung (110) eine Einkammer-Reinigungsvorrichtung (120, 122, 240) mit genau einer Reinigungskammer (118) aufweist, wobei die Reinigungskammer (118) zur Eingabe und Entnahme des Reinigungsguts (112) geöffnet und verschlossen werden kann, wobei die Einkammer-Reinigungsvorrichtung (120, 122, 240) ein Reinigungs- und Desinfektionsgerät (240) zur Reinigung von Pflegeutensilien aufweist, wobei die Pflegeutensilien zur Aufnahme flüssiger und/oder fester menschlicher Ausscheidungen von mindestens 100 ml geeignet sind, wobei das Reinigungs- und Desinfektionsgerät (240) mindestens einen Abfluss (154) zur Entsorgung von in den Pflegeutensilien enthaltenen Ausscheidungen aufweist, wobei der Abfluss (154) mit einem Geruchsverschluss (156) ausgestaltet ist, wobei die Reinigungsvorrichtung (110) eingerichtet ist, um das Reaktivgas aus der Reinigungskammer (118) in den Abfluss (154) hinter den Geruchsverschluss (156) abzuführen, wobei die Reinigungsvorrichtung (110) eingerichtet ist, um das Reaktivgas nach Kontakt mit zumindest einem Teil des Reinigungsguts (112) durch mindestens eine Aufbereitungsvorrichtung (234) zu leiten, insbesondere mindestens einen Filter (236) und/oder mindestens einen Katalysator (238).

2. Reinigungsvorrichtung (110) nach dem vorhergehenden Anspruch, wobei Reinigungsvorrichtung (110) mindestens eine Ansteuerung (126) aufweist, wobei die Ansteuerung (126) eingerichtet ist, um mindestens ein Reinigungsprogramm in der Reinigungsvorrichtung (110) zu steuern, wobei in mindestens einem Reinigungsprogrammschritt das Reinigungsgut (112) mit dem Reinigungsfluid beaufschlagt wird und wobei in mindestens einem Desinfektionsschritt das Reinigungsgut (112) mit dem Reaktivgas beaufschlagt wird, vorzugsweise nach Durchführung des Reinigungsprogrammschritts.

3. Reinigungsvorrichtung (110) nach einem der vorhergehenden Ansprüche, wobei die Reinigungsvorrichtung (110) mindestens eine Druckvorrichtung (172) aufweist, wobei die Druckvorrichtung (172) eingerichtet ist, um einen Überdruck und/oder einen Unterdruck in mindestens einem Teil der Reinigungsvorrichtung (110) zu erzeugen, wobei die Druckvorrichtung (172) eingerichtet ist, um das Reaktivgas nach Kontakt mit zumindest einem Teil der Reinigungsvorrichtung (110) in den mindestens einen Abfluss (154) abzuleiten.

4. Reinigungsvorrichtung (110) nach einem der vorhergehenden Ansprüche, wobei die Plasmaquelle (162, 164, 214, 218, 246, 248, 278) derart ausgestaltet ist, dass das Reaktivgas mit zumindest einem Teil der Fluidquelle (128) in Kontakt gebracht wird.

5. Reinigungsvorrichtung (110) nach einem der vorhergehenden Ansprüche, wobei die Plasmaquelle (162, 164, 214, 218, 246, 248, 278) auf mindestens eine der folgenden Arten ausgestaltet ist: die Plasmaquelle (162, 164, 214, 218, 246, 248, 278) ist innerhalb der Reinigungskammer (118) angeordnet und eingerichtet, um das Plasma innerhalb der Reinigungskammer (118) zu zünden; die Plasmaquelle (162, 164, 214, 218, 246, 248, 278) ist außerhalb der Reinigungskammer (118) angeordnet, und die Reinigungsvorrichtung (110) ist eingerichtet, um das Reaktivgas in zumindest einen Teil der Reinigungskammer (118) zu leiten, insbesondere durch ein Gebläse (170, 220), eine Saugvorrichtung oder eine Überdruckvorrichtung.

6. Reinigungsvorrichtung (110) nach einem der vorhergehenden Ansprüche, wobei das Reaktivgas ein Plasma umfasst, insbesondere ein kaltes Plasma mit einer Temperatur von weniger als 100 °C, vorzugsweise von weniger als 80 °C und besonders bevorzugt von weniger als 60 °C.

7. Reinigungsvorrichtung (110) nach einem der vorhergehenden Ansprüche, wobei das Reaktivgas mindestens eine durch das Plasma erzeugbare Verbindung aufweist, ausgewählt aus der Gruppe bestehend aus: Ozon; atomarem Sauerstoff, atomarem Stickstoff; Wasserstoffperoxid.

8. Reinigungsvorrichtung (110) nach einem der vorhergehenden Ansprüche, wobei die Pflegeutensilien zur Aufnahme menschlicher Ausscheidungen von mindestens 500 ml geeignet sind.

9. Reinigungsvorrichtung (110) nach einem der vorhergehenden Ansprüche, wobei der Abfluss (154) zur Entsorgung von in den Pflegeutensilien enthaltenen Ausscheidungen mit Flüssigkeitsmengen von mindestens 500 ml eingerichtet ist.

10. Reinigungsvorrichtung (110) nach einem der vorhergehenden Ansprüche, wobei der Geruchsverschluss (156) mindestens einen Siphonbogen (158) aufweist.

11. Reinigungsvorrichtung (110) nach einem der vorhergehenden Ansprüche, wobei der Abfluss (154) einen Durchmesser oder Äquivalentdurchmesser von mindestens 30 mm aufweist.

12. Reinigungsvorrichtung (110) nach einem der vorhergehenden Ansprüche, wobei der Abfluss (154) einen Durchmesser oder Äquivalentdurchmesser von mindestens 50 mm aufweist.

13. Verfahren zur Reinigung von Reinigungsgut (112), wobei das Reinigungsgut (112) in mindestens einer Reinigungskammer (118) mit mindestens einem Reinigungsfluid beaufschlagt wird, wobei weiterhin mittels mindestens einer Plasmaquelle (162, 164, 214, 218, 246, 248, 278) ein Plasma in mindestens einem Gas gezündet und mindestens ein Reaktivgas erzeugt wird, wobei das Reaktivgas mit zumindest einem Teil des Reinigungsguts (112) in Kontakt gebracht wird, wobei die Reinigungsvorrichtung (110) nach einem der vorhergehenden Ansprüche verwendet wird.

## Claims

1. Cleaning apparatus (110) for cleaning items to be cleaned (112), wherein the cleaning apparatus (110) comprises a cleaning chamber (118) for receiving the items to be cleaned (112), wherein the cleaning apparatus (110) comprises, in the cleaning chamber (118), at least one fluid source (128) for subjecting the items to be cleaned (112) to the action of at least one cleaning fluid, wherein the cleaning apparatus (110) further comprises at least one plasma source (162, 164, 214, 218, 246, 248, 278), wherein the plasma source (162, 164, 214, 218, 246, 248, 278) is designed to ignite at least one plasma in at least one gas and to generate at least one reactive gas, wherein the cleaning apparatus (110) is designed to bring the reactive gas into contact with at least part of the items to be cleaned (112), wherein the cleaning apparatus (110) comprises a one-chamber cleaning apparatus (120, 122, 240) having exactly one cleaning chamber (118), wherein the cleaning chamber (118) can be opened and closed in order to input and remove the items to be cleaned (112), wherein the one-chamber cleaning apparatus (120, 122, 240) comprises a cleaning and disinfection unit (240) for cleaning care utensils, wherein the care utensils are suitable for receiving liquid and/or solid human excretions of at least 100 ml, wherein the cleaning and disinfection unit (240) comprises at least one drain (154) for the disposal of excretions contained in the care utensils, wherein the drain (154) is formed with an odour trap (156), wherein the cleaning apparatus (110) is designed to discharge the reactive gas from the cleaning chamber (118) into the drain (154) after the odour trap (156), wherein the cleaning apparatus (110) is designed to convey the reactive gas, after contact with at least part of the items to be cleaned (112), through at least one preparation apparatus (234), in particular at least one filter (236) and/or at least one catalyst (238).

2. Cleaning apparatus (110) according to the preceding claim, wherein the cleaning apparatus (110) comprises at least one controller (126), wherein the controller (126) is designed to control at least one cleaning program in the cleaning apparatus (110), wherein the items to be cleaned (112) are acted on by the cleaning fluid in at least one cleaning program step, and wherein the items to be cleaned (112) are acted on by the reactive gas in at least one disinfection step, preferably after the cleaning program step has been carried out.

3. Cleaning apparatus (110) according to one of the preceding claims, wherein the cleaning apparatus (110) comprises at least one pressure apparatus (172), wherein the pressure apparatus (172) is designed to generate an overpressure and/or a negative pressure in at least part of the cleaning apparatus (110), wherein the pressure apparatus (172) is designed to divert the reactive gas, after contact with at least part of the cleaning apparatus (110), into the at least one drain (154).

4. Cleaning apparatus (110) according to one of the preceding claims, wherein the plasma source (162, 164, 214, 218, 246, 248, 278) is formed in such a way that the reactive gas is brought into contact with at least part of the fluid source (128).

5. Cleaning apparatus (110) according to one of the preceding claims, wherein the plasma source (162, 164, 214, 218, 246, 248, 278) is formed in at least one of the following ways: the plasma source (162, 164, 214, 218, 246, 248, 278) is arranged inside the cleaning chamber (118) and is designed to ignite the plasma inside the cleaning chamber (118); the plasma source (162, 164, 214, 218, 246, 248, 278) is arranged outside the cleaning chamber (118), and the cleaning apparatus (110) is designed to convey the reactive gas into at least part of the cleaning chamber (118), in particular by means of a fan (170, 220), a suction apparatus or an overpressure apparatus.

6. Cleaning apparatus (110) according to one of the preceding claims, wherein the reactive gas comprises a plasma, in particular a cold plasma having a temperature of less than 100°C, preferably of less than 80°C, and particularly preferably of less than 60°C.

7. Cleaning apparatus (110) according to one of the preceding claims, wherein the reactive gas comprises at least one compound which can be generated by the plasma, selected from the group consisting of: ozone; atomic oxygen, atomic nitrogen; and hydrogen peroxide.

8. Cleaning apparatus (110) according to one of the preceding claims, wherein the care utensils are suitable for receiving human excretions of at least 500 ml.

9. Cleaning apparatus (110) according to one of the preceding claims, wherein the drain (154) is designed for the disposal of excretions contained in the care utensils with liquid volumes of at least 500 ml.

10. Cleaning apparatus (110) according to one of the preceding claims, wherein the odour trap (156) comprises at least one siphon bend (158).

11. Cleaning apparatus (110) according to one of the preceding claims, wherein the drain (154) has a diameter or equivalent diameter of at least 30 mm.

12. Cleaning apparatus (110) according to one of the preceding claims, wherein the drain (154) has a diameter or equivalent diameter of at least 50 mm.

13. Method for cleaning items to be cleaned (112), wherein the items to be cleaned (112) are acted on in at least one cleaning chamber (118) by at least one cleaning fluid, wherein, furthermore, a plasma is ignited in at least one gas by means of at least one plasma source (162, 164, 214, 218, 246, 248, 278) and at least one reactive gas is generated, wherein the reactive gas is brought into contact with at least part of the items to be cleaned (112), wherein the cleaning apparatus (110) according to one of the preceding claims is used.

## Revendications

1. Dispositif de nettoyage (110) destiné à nettoyer des articles à nettoyer (112), le dispositif de nettoyage (110) comportant une chambre de nettoyage (118) destinée à recevoir les articles à nettoyer (112), le dispositif de nettoyage (110) comportant dans la chambre de nettoyage (118) au moins une source de fluide (128) destiné à soumettre un article à nettoyer (112) à au moins un fluide de nettoyage, le dispositif de nettoyage (110) comprenant en outre au moins une source de plasma (162, 164, 214, 218, 246, 248, 278), la source de plasma (162, 164, 214, 218, 246, 248, 278) étant conçue pour allumer au moins un plasma dans au moins un gaz et pour générer au moins un gaz réactif, le dispositif de nettoyage (110) étant conçu pour mettre le gaz réactif en contact avec au moins une partie des articles à nettoyer (112), le dispositif de nettoyage (110) comportant un dispositif de nettoyage à chambre unique (120, 122, 240) pourvu d'exactement une chambre de nettoyage (118), la chambre de nettoyage (118) pouvant être ouverte et fermée pour l'entrée et le retrait des articles à nettoyer (112), le dispositif de nettoyage à chambre unique (120, 122, 240) comportant un dispositif de nettoyage et de désinfection (240) destiné à nettoyer les ustensiles de soins, les ustensiles de soins étant aptes à recevoir des excrétions humaines liquides et/ou solides d'au moins 100 ml, le dispositif de nettoyage et de désinfection (240) comportant au moins un drain (154) destiné à l'évacuation d'excrétions contenues dans les ustensiles de soins, le drain (154) étant muni d'une fermeture anti-odeurs (156), le dispositif de nettoyage (110) étant conçu pour évacuer le gaz réactif de la chambre de nettoyage (118) jusque dans le drain (154) situé derrière la fermeture anti-odeurs (156), le dispositif de nettoyage (110) étant conçu pour guider le gaz réactif à travers au moins un dispositif de traitement (234) après contact avec au moins une partie des articles à nettoyer (112), en particulier au moins un filtre (236) et/ou au moins un catalyseur (238).

2. Dispositif de nettoyage (110) selon la revendication précédente, le dispositif de nettoyage (110) comportant au moins une commande (126), la commande (126) étant conçue pour commander au moins un programme de nettoyage dans le dispositif de nettoyage (110), les articles à nettoyer (112) étant soumis au fluide de nettoyage dans au moins une étape de programme de nettoyage, et les articles à nettoyer (112) étant soumis au gaz réactif dans au moins une étape de désinfection, de préférence après la réalisation de l'étape de programme de nettoyage.

3. Dispositif de nettoyage (110) selon l'une des revendications précédentes, le dispositif de nettoyage (110) comportant au moins un dispositif de pression (172), le dispositif de pression (172) étant conçu pour générer une surpression et/ou une dépression dans au moins une partie du dispositif de nettoyage (110), le dispositif de pression (172) étant conçu pour évacuer le gaz réactif jusque dans l'au moins un drain (154) après contact avec au moins une partie du dispositif de nettoyage (110).

4. Dispositif de nettoyage (110) selon l'une des revendications précédentes, la source de plasma (162, 164, 214, 218, 246, 248, 278) étant conçue de telle sorte que le gaz réactif soit mis en contact avec au moins une partie de la source de fluide (128).

5. Dispositif de nettoyage (110) selon l'une des revendications précédentes, la source de plasma (162, 164, 214, 218, 246, 248, 278) étant conçue selon l'une au moins des manières suivantes : la source de plasma (162, 164, 214, 218, 246, 248, 278) est disposée à l'intérieur de la chambre de nettoyage (118) et est conçue pour allumer le plasma à l'intérieur de la chambre de nettoyage (118) ; la source de plasma (162, 164, 214, 218, 246, 248, 278) est disposée à l'extérieur de la chambre de nettoyage (118) et le dispositif de nettoyage (110) est conçu pour guider le gaz réactif jusque dans au moins une partie de la chambre de nettoyage (118), notamment par une soufflante (170, 220), un dispositif d'aspiration ou un dispositif de surpression.

6. Dispositif de nettoyage (110) selon l'une des revendications précédentes, le gaz réactif comprenant un plasma, notamment un plasma froid à une température inférieure à 100 °C, de préférence inférieure à 80 °C et de manière particulièrement préférée inférieure à 60 °C.

7. Dispositif de nettoyage (110) selon l'une des revendications précédentes, le gaz réactif comportant au moins un composé qui peut être généré par le plasma et qui est choisi dans le groupe comprenant : l'ozone ; l'oxygène atomique, l'azote atomique; le peroxyde d'hydrogène.

8. Dispositif de nettoyage (110) selon l'une des revendications précédentes, les ustensiles de soins étant aptes à recevoir des excrétions humaines d'au moins 500 ml.

9. Dispositif de nettoyage (110) selon l'une des revendications précédentes, le drain (154) étant conçu pour évacuer des excrétions contenues dans les ustensiles de soins avec des quantités de liquide d'au moins 500 ml.

10. Dispositif de nettoyage (110) selon l'une des revendications précédentes, la fermeture anti-odeurs (156) comportant au moins un coude de siphon (158).

11. Dispositif de nettoyage (110) selon l'une des revendications précédentes, le drain (154) ayant un diamètre ou un diamètre équivalent d'au moins 30 mm.

12. Dispositif de nettoyage (110) selon l'une des revendications précédentes, le drain (154) ayant un diamètre ou un diamètre équivalent d'au moins 50 mm.

13. Procédé de nettoyage des articles à nettoyer (112), les articles à nettoyer (112) étant soumis à au moins un fluide de nettoyage dans au moins une chambre de nettoyage (118), un plasma étant allumé au moyen d'au moins une source de plasma (162, 164, 214, 218, 246, 248, 278) dans au moins un gaz et au moins un gaz réactif étant généré, le gaz réactif étant mis en contact avec au moins une partie des articles à nettoyer (112), le dispositif de nettoyage (110) étant utilisé selon l'une des revendications précédentes.
